# EUROPEAN PATENT SPECIFICATION

(11) **EP 2 504 386 B1**
(45) Date of publication and mention of the grant of the patent: **22.03.2017**
(21) Application number: 10782476.5
(22) Date of filing: 09.11.2010
(51) Int. Cl.: C08K 5/00, C08L 33/06, A61K 8/04

(54) **SURFACTANT-POLYMER BLENDS**
TENSID-POLYMER MISCHUNGEN
MÉLANGES POLYMÈRE/TENSIOACTIFS

(30) Priority: 23.11.2009 US 263436 P
(43) Date of publication of application: 03.10.2012
(73) Proprietor: Lubrizol Advanced Materials, Inc., Cleveland, OH 44141-3247 (US)
(72) Inventor: GALLEGUILLOS, Ramiro, Hudson Ohio 44236 (US); MULLEE, James E., Garrettsville Ohio 44231 (US); PUROHIT, Pinky G., Beachwood Ohio 44122 (US)
(74) Representative: dompatent von Kreisler Selting Werner - Partnerschaft von Patent- und Rechtsanwälten mbB
(86) International application number: PCT/US2010/055962
(87) International publication number: WO 2011/062805

(56) References cited:
- WO-A1-03/062288
- WO-A1-2006/091189

## Description

### FIELD OF THE INVENTION

The present invention relates to surfactant-polymer blends that are useful as thickeners for a variety of health care, home care, institutional and industrial (collectively "I&I") care products. In one embodiment, the surfactant-polymer blends of the present invention comprises: (a) at least one anionic surfactant; (b) at least one amphoteric or zwitterionic surfactant; and (c) at least one hydrophobically-modified polymer formed from a mixture comprising: (i) an α,β-ethylenically unsaturated carboxylic acid monomer; (ii) a nonionic, co-polymerizable α,β-ethylenically unsaturated monomer; and (iii) an ethylenically unsaturated hydrophobically-modified associative monomer. In another embodiment, the surfactant-polymer blends of the present invention comprises: (a) at least one anionic surfactant; (b) at least one amphoteric or zwitterionic surfactant; and (c) at least one hydrophobically-modified polymer formed from a mixture comprising: (i) an α,β-ethylenically unsaturated carboxylic acid monomer; (ii) a nonionic, co-polymerizable α,β-ethylenically unsaturated monomer; and (iii) an ethylenically unsaturated hydrophobically-modified associative monomer; and (d) at least one salt. The surfactant-polymer blends of the present invention are useful as thickeners in a variety of products including, but not limited to, personal care products, health care products, household care products, institutional and industrial care products, and industrial applications.

### BACKGROUND OF THE INVENTION

Rheology modifiers, or thickeners, have several roles in aqueous systems. They increase viscosity and maintain viscosity at required levels under specified processing conditions and end use situations. In cosmetics and personal care items, for example, the thickener improves body, smoothness and silkiness, making the product more aesthetically pleasing.

Many rheology modifiers, both natural and synthetic, are known. Natural rheology modifiers include, for example, casein, alginates, gum tragacanth, and modified cellulose, including methyl cellulose, hydroxyethyl cellulose, hydroxypropyl cellulose and carbomethoxy cellulose. These natural products vary in their thickening efficiency and generally provide poor flow and leveling properties; they are also subject to microbial attack and require the additional presence of antimicrobial agents. Synthetic rheology modifiers include various acrylic polymers and maleic anhydride copolymers. Some of these are found to be pH dependent, others are hydrolytically unstable, some require large amounts of thickener to effectively increase viscosity, others are sensitive to various components normally found in aqueous systems, and still others require large amounts of salt to increase the viscosity to a desired or suitable level.

Additionally, many cleansing formulations are prepared using mixtures or combination of surfactants and as a result are, or can be, very difficult to thicken. Although some surfactant mixtures can be thickened with a salt compound alone, this method is not satisfactory for instances as may cleansing formulations are difficult, if not impossible, to thicken with a salt compound even if said salt compound is employed at a high concentration level.

Furthermore, many surfactant formulations such as shampoos, body washes, shower gels, or so-called facial soaps are prepared using high levels of zwitterionic or amphoteric surfactants, commonly called betaines. Although, some of these formulations can be thickened with a salt compound some cannot. For example, surfactant compositions with less than about 3 weight percent of one or more amphoteric or zwitterionic surfactants, or even less than about 2 weight percent, are impossible to thicken with a salt compound. Although many polymeric thickeners exist that can thicken formulations having low betaine levels such polymeric thickeners must be used at very high levels. This results in mucous-like fluids that possess very poor flow properties at room temperature in addition to possessing very poor shelf lives. This also results in very poor flow properties at both high and low temperatures. Other thickeners such as so-called micellar thickeners or non-ionic thickeners also result in similar poor formulation flow properties. In most cases, formulations thickened as described above lose their desirable viscosity completely at about 30°C (i.e., a "warm summer day"), thereby resulting in a runny liquid with poor performance and aesthetic characteristics.

WO 2007/111963 discloses in example 12 a personal care product containing 1.5 % sodium trideceth sulfate, 1.5 % sodium myristoyl sarcosinate, 3.0 % sodium lauroamphoacetate, 0.5 % acrylates/beheneth-25 methacylate copolymer and citric acid to adjust the pH of the composition to 5.5 to 6.

Given the above, there is a need in the art for surfactant-polymer blends that function in combination with a wide variety of surfactants and do not need a high level, or amount, of salt to function as useful thickeners in a variety of personal care products. Also, there is an additional need in the art for a thickening system that provides superior flow properties and flow aesthetics at low and/or high temperature (5°C to 70°C).

### SUMMARY OF THE INVENTION

The present invention relates to surfactant-polymer blends that are useful as thickeners for a variety of personal care products.

In particular, the present invention relates to a surfactant-polymer blend comprising: A surfactant-polymer blend comprising:
(a) at least one anionic surfactant;
(b) at least one amphoteric or zwitterionic surfactant;
(c) at least one hydrophobically-modified polymer formed from a mixture comprising:
   (i) at least one α,β-ethylenically unsaturated carboxylic acid monomer selected from acrylic acids, methacrylic acids, crotonic acids, acyloxypropionic acids, maleic acids, fumaric acids, itaconic acids, aconitic acids, half- or mono-esters of dibasic or tribasic acids;
   (ii) at least one nonionic, co-polymerizable α,β-ethylenically unsaturated monomer selected from a C1 to C4 alkyl acrylate, C1 to C4 alkyl methacrylate; and
   (iii) at least one ethylenically unsaturated hydrophobically-modified associative monomer selected from cetyl polyethoxylated (meth)acrylate (CEM), cetearyl polyethoxylated (meth)acrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, lacceryl polyethoxylated (meth)acrylate, nonylphenol polyethoxylated (meth)acrylate (NEM), tristyrylphenol polyethoxylated (meth)acrylate (TEM), palmyl polyethoxylated itaconate (PEI), stearyl polyethoxylated itaconate (SEI), behenyl polyethoxylated itaconate (BEI), palmyl polyethoxylated maleate (PEML), stearyl polyethoxylated maleate (SEM), behenyl polyethoxylated maleate (BEML), palmyl polyethoxylated allyl ether (PEAE), stearyl polyethoxylated allyl ether (SEAE), behenyl polyethoxylated allyl ether (BEAE), wherein the polyethoxylated portion of the foregoing monomers contain from 3 to 200 ethylene oxide repeating unit; and
(d) at least one monovalent salt selected from lithium chloride, sodium chloride, ammonium chloride, potassium chloride, sodium benzoate, sodium salicylate or mixtures of any two or more thereof.

The surfactant-polymer blends of the present invention are useful as thickeners in a variety of products including, but not limited to, personal care products, health care products, household care products, institutional and industrial care products, and industrial applications.

### DETAILED DESCRIPTION OF THE INVENTION

The present invention relates to surfactant-polymer blends mentioned above.

In one embodiment, the surfactant-polymer blend of the present invention is a linear, non-crosslinked, hydrophobically-modified polymethacrylic acid polyelectrolyte polymer. In one embodiment, the surfactant-polymer blends of the present invention possess superior thickening efficiency in a cleansing composition. Furthermore, the surfactant-polymer blends of the present invention possess superior thickening efficiency even in the presence of moderate amounts of electrolytes (including monovalent electrolytes) such as NaCl, KCI and other salts.

As used herein, "moderate amounts of electrolytes" means that the amount of electrolyte, or electrolytes, present in a composition is less than 4 weight percent, less than 3 weight percent, less than 2 weight percent, less than 1.5 weight percent, or even less than 1 weight percent, based on the total amount of the composition.

In one embodiment, the weight ratio of the combination of components (a) and (b) (the at least one anionic surfactant and the at least one amphoteric or zwitterionic surfactant, respectively) to component (c) (the at least one hydrophobically-modified polymer) is in the range of from 2500:1 to 5:1, or from 2250:1 to 7.5:1, or from 2000:1 to 10:1, or from 1750:1 to 15:1, or from 1500:1 to 20:1, or from 1250:1 to 25:1, or from 1000:1 to 30:1, or from 750:1 to 50:1, or from 500:1 to 75:1, or even from 250:1. to 100:1.

In still another embodiment, the weight ratio of the combination of components (a) and (b) (the at least one anionic surfactant and the at least one amphoteric or zwitterionic surfactant, respectively) to component (c) (the at least one hydrophobically-modified polymer) is in the range of from 100:1 to 1:1, or from 75:1 to 2.5:1, or from 50:1 to 5:1, or from 40:1 to 7.5:1, or from 30:1 to 10:1, or from 25:1 to 12.5:1, or even from 20:1 to 15:1. Here, as well as elsewhere in the specification and claims, individual numerical values, or limits, can be combined to form additional non-disclosed and/or non-stated ranges.

In one embodiment, the weight ratio of the combination of components (a) and (b) (the at least one anionic surfactant and the at least one amphoteric or zwitterionic surfactant, respectively) to component (c) (the at least one hydrophobically-modified polymer) to component (d) (the at least one salt) is in the range of from 2500:1:1 to 5:1:1, or from 2250:1:1 to 7.5:1:1, or from 2000:1:1 to 10:1:1, or from 1750:1:1 to 15:1:1, or from 1500:1:1 to 20:1:1, or from 1250:1:1 to 25:1:1, or from 1000:1:1 to 30:1:1, or from 750:1:1 to 50:1:1, or from 500:1:1 to 75:1:1, or even from 250:1:1 to 100:1:1.

In still another embodiment, the weight ratio of the combination of components (a) and (b) (the at least one anionic surfactant and the at least one amphoteric or zwitterionic surfactant, respectively) to component (c) (the at least one hydrophobically-modified polymer) to component (d) (the at least one salt) is in the range of from 100:1:1 to 1:1:1, or from 75:1:1 to 2.5:1:1, or from 50:1:1 to 5:1:1, or from 40:1:1 to 7.5:1:1, or from 30:1:1 to 10:1:1, or from 25:1:1 to 12.5:1:1, or even from 20:1:1 to 15:11.1.

In one embodiment, a surfactant-polymer blend of the present invention can be added to one or more aqueous compositions (e.g., personal care compositions) as a thickener. In one instance, a surfactant-polymer blend of this embodiment can be added at a concentration of 0.1 weight percent to 50 weight percent, based on 100 weight percent of the overall aqueous composition. In another embodiment, a surfactant-polymer blend of this embodiment can be added at a concentration of 0.5 weight percent to 45 weight percent, or from 1 weight percent to 40 weight percent, or from 5 weight percent to 35 weight percent, or from 10 weight percent to 30 weight percent, or even from 15 weight percent to 25 weight percent.

In one embodiment, a surfactant-polymer-salt blend of the present invention can be added to one or more aqueous compositions (e.g., personal care compositions) as a thickener. In one instance, a surfactant-polymer-salt blend of this embodiment can be added at a concentration of 0.1 weight percent to 50 weight percent, based on 100 weight percent of the overall aqueous composition. In another embodiment, a surfactant-polymer blend of this embodiment can be added at a concentration of 0.5 weight percent to 45 weight percent, or from 1 weight percent to 40 weight percent, or from 5 weight percent to 35 weight percent, or from 10 weight percent to 30 weight percent, or even from 15 weight percent to 25 weight percent.

In one embodiment, a surfactant-polymer blend of the present invention can be added to one or more aqueous compositions (e.g., personal care compositions) as a thickener at any concentration and/or weight range to yield a viscosity of at least 4,000 mPa·s (as measured after 24 hours with a Brookfield RVT viscometer at 20 rpm, 25°C - Spindle #4). In another embodiment, a surfactant-polymer blend of this embodiment can be added at any concentration and/or weight range to yield a viscosity of at least 5,000 mPa·s, at least 6,000 mPa·s, at least 7,500 mPa·s, at least 10,000 mPa·s, at least 12,500 mPa·s, at least 15,000 mPa·s, at least 17,500 mPa·s, at least 20,000 mPa·s, at least 25,000 mPa·s, at least 30,000 mPa·s, at least 35,000 mPa·s, at least 40,000 mPa·s, at least 45,000 mPa·s, at least 50,000 mPa·s, at least 55,000 mPa·s, at least 60,000 mPa·s, at least 65,000 mPa·s, at least 70,000 mPa·s, at least 75,000 mPa·s, at least 80,000 mPa·s, at least 85,000 mPa·s, at least 90,000 mPa·s, at least 95,000 mPa·s, or even at least 100,000 mPa·s.

In one embodiment, a surfactant-polymer-salt blend of the present invention can be added to one or more aqueous compositions (e.g., personal care compositions) as a thickener at any concentration and/or weight range to yield a viscosity of at least 4,000 mPa·s (as measured after 24 hours with a Brookfield RVT viscometer at 20 rpm, 25°C - Spindle #4). In another embodiment, a surfactant-polymer-salt blend of this embodiment can be added at any concentration and/or weight range to yield a viscosity of at least 5,000 mPa·s, at least 6,000 mPa·s, at least 7,500 mPa·s, at least 10,000 mPa·s, at least 12,500 mPa·s, at least 15,000 mPa·s, at least 17,500 mPa·s, at least 20,000 mPa·s, at least 25,000 mPa·s, at least 30,000 mPa·s, at least 35,000 mPa·s, at least 40,000 mPa·s, at least 45,000 mPa·s, at least 50,000 mPa·s, at least 55,000 mPa·s, at least 60,000 mPa·s, at least 65,000 mPa·s, at least 70,000 mPa·s, at least 75,000 mPa·s, at least 80,000 mPa·s, at least 85,000 mPa·s, at least 90,000 mPa·s, at least 95,000 mPa·s, or even at least 100,000 mPa·s. ranges.

As is discussed above, the surfactant-polymer blends and/or the surfactant-polymer-salt blends of the present invention can be added to any aqueous composition that are desired to be thickened. Such aqueous compositions include, but are not limited to, personal care products, health care products, institutional and industrial care products, and industrial applications. It should also be noted that although one or more salt compounds are not specifically added to various surfactant-polymer blends of the present invention some of the surfactant-polymer blend embodiments of the present invention may still contain one or more salts therein. In one instance, such one or more salt compounds can exist as minor components of various surfactant compositions that are utilized in conjunction with the various surfactant-polymer blend compositions of the present invention.

Accordingly, in one embodiment, the surfactant-polymer and/or surfactant-polymer-salt blends of the present invention have less than 10 weight percent salt, less than 7.5 weight percent salt, less than 5 weight percent salt, less than 2.5 weight percent salt, less than 1 weight percent salt, less than 0.5 weight percent salt, less than 0.1 weight percent salt, less than 0.01 weight percent salt, less than 0.001 weight percent salt, less than 0.0001 weight percent salt, less than 0.00001 weight percent salt, or even 0 weight percent salt (i.e., "salt-free"), based on the total weight of the surfactant-polymer and/or surfactant-polymer-salt blend. As would be apparent to those of skill in the art, when a blend of the present invention is free from added salt, such a blend is referred to herein as a surfactant-polymer blend. This does not mean however that all surfactant-polymer blends of the present invention are salt-free as defined above due to the possibility of one or more salt compounds being present in minor amounts in the various surfactants utilized in conjunction with the present invention.

The term "personal care products" as used herein includes, without being limited thereto, cosmetics, toiletries, cosmeceuticals and beauty aids, personal hygiene and cleansing products applied to the skin, hair, scalp, and nails of humans and animals. The term "health care products" as used herein includes, without being limited thereto, pharmaceuticals, pharmacosmetics, oral care products (mouth, teeth), eye care products, ear care products and over-the-counter products and appliances, such as patches, plasters, dressings and the like, and medical devices externally applied to or into the body of humans and animals for ameliorating a health-related or medical condition, for generally maintaining hygiene or well-being, and the like. The term "body" includes the keratinous (hair, nails) and non-keratinous skin areas of the entire body (face, trunk, limbs, hands and feet), the tissues of body openings and eyes, and the term "skin" includes the scalp and mucous membranes. The term "household care products" as used herein includes, without being limited thereto, products employed in a domestic household for surface cleaning or biocidal cleaning products for maintaining sanitary conditions, such as in the kitchen and bathroom, and laundry products for fabric care and cleaning, and the like. The term "institutional and industrial care" and "I&I," as used herein includes, without being limited thereto, products employed for cleaning or maintaining sanitary conditions in industrial and institutional environments, including hospital and health care facilities, and the like.

As discussed above, in one embodiment the surfactant-polymer blends of the present invention comprises: (a) the at least one anionic surfactant; (b) the at least one amphoteric or zwitterionic surfactant; and (c) the at least one hydrophobically-modified polymer formed from a mixture comprising: (i) the α,β-ethylenically unsaturated carboxylic acid monomer; (ii) the nonionic, co-polymerizable α,β ethylenically unsaturated monomer; and (iii) the ethylenically unsaturated hydrophobically-modified associative monomer. In another embodiment, the surfactant-polymer blends of the present invention comprises: (a) the at least one anionic surfactant; (b) the at least one amphoteric or zwitterionic surfactant; and (c) at least one hydrophobically-modified polymer formed from a mixture comprising: (i) the α,β-ethylenically unsaturated carboxylic acid monomer; (ii) the nonionic, co-polymerizable α,β-ethylenically unsaturated monomer; and (iii) the ethylenically unsaturated hydrophobically-modified associative monomer; and (d) the at least one salt. The blends of this embodiment are referred to herein as surfactant-polymer-salt blends. The details of each component of the blends of the present invention will be discussed below.

In still yet another embodiment, the weight ratio of the combination of components (a) and (b) (the at least one anionic surfactant and the at least one amphoteric or zwitterionic surfactant, respectively) to component (c) (the at least one hydrophobically-modfied polymer) is in the range of from 40:1 to 10:1, where the ratio is calculated based on the amount of active matter contained in components (a), (b) and (c) (where the term "active matter" is as defined below). In still yet another embodiment, the weight ratio of the combination of components (a) and (b) (the at least one anionic surfactant and the at least one amphoteric or zwitterionic surfactant, respectively) to component (c) (the at least one hydrophobically-modified polymer) to component (d) (the at least one salt) is in the range of from 400:10:1 to 100:1:2, or even from 40:1:1 to 50:5:6, where the ratio is calculated based on the amount of active matter contained in components (a), (b) and (c) (where the term "active matter" is as defined below).

### Surfactant Components (a) and (b):

In one embodiment, the surfactant components (components (a) and (b)) of the present invention are selected from one or more anionic (component (a)) and one or more amphoteric or zwitterionic surfactant. In another embodiment, additional surfactants selected from various non-ionic, cationic and/or amide surfactants can be utilized in addition to the aforementioned surfactant components, components (a) and (b) discussed above. In another embodiment, any suitable combination of two or more of the aforementioned surfactants can be utilized as the surfactant component in the blends of the present invention.

### Anionic Surfactant-Component (a) :

In one embodiment, component (a) of the blends of the present invention can be selected from one or more suitable anionic surfactants. Such suitable anionic surfactants include, but are not limited to, one or more anionic cleansing surfactants which are cosmetically acceptable and suitable for topical application to the hair. Examples of suitable anionic cleansing surfactants include, but are not limited to, alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof (e.g., the sodium, magnesium, ammonium and mono-, di- and triethanolamine salts), or suitable mixtures of any two or more thereof. The alkyl and acyl groups contain, in one embodiment, from 8 to 18 carbon atoms, or even from 10 to 16 carbon atoms. In one embodiment, the alkyl and acyl groups can be unsaturated. The alkyl ether sulphates, alkyl ether sulphosuccinates, alkyl ether phosphates and alkyl ether carboxylic acids and salts thereof can contain from 1 to 20, or even from 1 to 10, ethylene oxide or propylene oxide units per molecule.

Typical anionic cleansing surfactants for use in personal care compositions (e.g., shampoo) of the invention include, but are not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

In another embodiment, suitable anionic cleansing surfactants for use in conjunction with the blends of the present invention include, but are not limited to, sodium lauryl sulphate, sodium lauryl ether sulphate(c)EO, (where c is from 1 to 3), sodium lauryl ether sulphosuccinate(c)EO, (where c is from 1 to 3), ammonium lauryl sulphate, ammonium lauryl ether sulphate(c)EO, (where c is from 1 to 3), sodium cocoyl isethionate and lauryl ether carboxylic acid(c) EO (where c is from 10 to 20), or suitable mixtures of any two or more thereof.

In personal care (e.g., shampoo) compositions the total amount of anionic cleansing surfactant generally ranges from 0.5 weight percent to 45 weight percent, or from 1.5 weight percent to 35 weight percent, or even from 5 weight percent to 20 weight percent, based on the total weight of the composition.

Shampoo compositions containing a blend according to one or more embodiments of the present invention will generally comprise one or more anionic cleansing surfactants which are cosmetically acceptable and suitable for topical application to the hair.

### Amphoteric or Zwitterionic Surfactants - Component (b):

In one embodiment, component (b) is selected from one or more amphoteric or zwitterionic surfactants such as alkylbetaines and alkylsulfobetaines are suitable for use in the blends of the present invention. In one embodiment, suitable amphoteric surfactants include, but are not limited to, cocoamidopropyl betaine, CAPB, cocobetaine, or mixtures of two or more thereof.

In another embodiment, a co-surfactant can be utilized. If present, the co-surfactant is either an amphoteric or zwitterionic surfactant which can be included in an amount ranging from 0.5 weight percent to 8 weight percent, or from 1 weight percent to 4 weight percent, based on the total weight of the composition to which a blend of the present invention is added.

Examples of suitable amphoteric or zwitterionic surfactants include, but not limited to, alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in personal care (e.g., shampoo) compositions include, but are not limited to, lauryl amine oxide, cocodimethyl sulphopropyl betaine, lauryl betaine, cocamidopropyl betaine, and sodium cocoamphoacetate. In another embodiment, suitable amphoteric or zwitterionic surfactants include, but are not limited to, cocoamidopropyl betaine, CAPB, cocobetaine, or mixture of two or more thereof.

In still another embodiment, mixtures of any of the foregoing amphoteric or zwitterionic surfactants can also be utilized in conjunction with the present invention. In still yet another embodiment, a mixture of cocamidopropyl betaine with additional amphoteric or zwitterionic surfactants as described above can be utilized. Another amphoteric or zwitterionic surfactant of interest is sodium cocoamphoacetate.

The total amount of surfactant (including any co-surfactant, and/or any emulsifier) in a shampoo composition of the invention is in the range of from 1 weight percent to 50 weight percent, or from 2 weight percent to 40 weight percent, or even from 10 weight percent to 25 weight percent, based on the total weight of the composition to which a blend of the present invention is added.

### Non-Ionic Surfactants and Co-surfactants:

In another embodiment, the surfactant portion of the blends of the present invention further comprises or more non-ionic surfactants and/or co-surfactants in addition to components (a) and (b) described above. Suitable non-ionic surfactants include, but are not limited to, alcohol ethoxylates, alkyl phenol ethoxylates, fatty acid alkanol amides, alkylamine oxides, N-methyl-glucamides, alkyl polyglucosides, or suitable mixtures of two or more thereof. In another embodiment, alcohol ethoxylate surfactants having hydrophilic-lipophilic balance (HLB) in the range about 2 to about 18.

In another embodiment, the blends of the present invention can further comprises one or more co-surfactants in order to help impart aesthetic, physical or cleansing properties to any composition to which a blend of the present invention is added. A non-limiting example of a co-surfactant is a non-ionic surfactant, which can be included in an amount ranging from 0.1 weight percent to 12 weight percent, or from 0.5 weight percent to 8 weight percent, or from 1 weight percent to 6 weight percent, or even from 2 weight percent to 5 weight percent, based on the total weight of the composition to which the blend of the present invention is added.

Suitable non-ionic surfactants for use in personal care formulations include, but are not limited to, one or more condensation products of aliphatic (C₈ to C₁₈) primary or secondary linear or branched chain alcohols or phenols with alkylene oxides, usually ethylene oxide and generally having from 2 to 30 ethylene oxide groups.

Other representative non-ionic surfactants include, but are not limited to, mono- or di-alkyl alkanolamides. Examples include coco mono- or diethanolamide and coco mono-isopropanolamide. Further non-ionic surfactants which can utilized in conjunction with shampoo compositions in accordance with the present invention include, but are not limited to, alkyl polyglycosides (APGs). Typically, an APG is a compound which comprises an alkyl group connected (optionally via a bridging group) to a block of one or more glycosyl groups. Suitable APGs can be defined by Formula (I):

RO-(G)ₐ (I)

wherein R is a linear or branched alkyl group, a linear or branched alkenyl group, or a linear or branched alkynyl group; and G is a saccharide group. In one embodiment, R can be a linear or branched alkyl group having from 3 to 30 carbon atoms, or a linear or branched alkyl group having from 5 to 25 carbon atoms, or a linear or branched alkyl group having from 7 to 20 carbon atoms, or even a linear or branched alkyl group having from 8 to 15 carbon atoms. In still another embodiment, R is a branched or straight chain alkyl group having from 9 to 13 carbon atoms. In another embodiment, R can be a linear or branched alkenyl group having from 3 to 30 carbon atoms, or a linear or branched alkenyl group having from 5 to 25 carbon atoms, or a linear or branched alkenyl group having from 7 to 20 carbon atoms, or even a linear or branched alkenyl group having from 8 to 15 carbon atoms. In still another embodiment, R is a branched or straight chain alkenyl group having from 9 to 13 carbon atoms.

In still another embodiment, R can be a linear or branched alkynyl group having from 3 to 30 carbon atoms, or a linear or branched alkynyl group having from 5 to 25 carbon atoms, or a linear or branched alkynyl group having from 7 to 20 carbon atoms, or even a linear or branched alkynyl group having from 8 to 15 carbon atoms. In still another embodiment R is a branched or straight chain alkynyl group having from 9 to 13 carbon atoms.

In one embodiment, G is selected from C₅ or C₆ monosaccharide residues, and is in one instance a glucoside. In another embodiment, G is selected from glucose, xylose, lactose, fructose, mannose, or derivatives thereof. In still another embodiment, G is glucose. In one embodiment, "a" is an integer in the range of from 1 to 10, or 2 to 8, or 3 to 7, or even from 4 to 6. In still another embodiment, "a" is in the range of from 1.1 to 2, or even from 1.3 to 1.5.

Suitable alkyl polyglycosides for use in the invention are commercially available and include, but are not limited to, materials identified as: Oramix NS10 ex Seppic; Plantaren 1200 and Plantaren 2000 ex from Henkel. Other sugar-derived nonionic surfactants which can be included in compositions of the invention include, but are not limited to, C₁₀ to C₁₈ N-alkyl (C₁ to C₆) polyhydroxy fatty acid amides, such as the C₁₂ to C₁₈N-methyl glucamides, as described for example in WO 92/06154 and United States Patent No. 5,194,639, and N-alkoxy polyhydroxy fatty acid amides, such as C₁₀ to C₁₈ N-(3-methoxypropyl) glucamide.

In another embodiment, suitable non-ionic surfactants include, but are not limited to, hydrophobically modified alkoxylated methyl glucoside, such as PEG 120 methyl glucose dioleate, PEG-120 methyl glucose trioleate, and PEG-20 methyl glucose sesquistearate, available from Lubrizol Advanced Materials, Inc., under the trade names, Glucamate^{®} DOE-120, Glucamate™ LT, and Glucamate™ SSE-20, respectively, are also suitable non-ionic surfactants. Other suitable hydrophobically modified alkoxylated methyl glucosides are disclosed in United States Patent Nos. 6.573.375 and 6,727,357.

### Cationic Surfactants:

In another embodiment, the surfactant portion of the blends of the present invention further comprises or more cationic surfactants in addition to components (a) and (b) described above. In one embodiment. personal care (e.g., conditioners) products that utilize a blend according to an embodiment of the present invention will typically comprise one or more cationic surfactants which are cosmetically acceptable and suitable for topical application to the hair or skin.

Examples of suitable cationic surfactants for use the blends of the present invention, where such blends are intended for use in personal care products (e.g., conditioners), include, but are not limited to, cetyltrimethylammonium chloride, behenyltrimethylammonium chloride, cetylpyridinium chloride, tetramethyl ammonium chloride, tetraethyl ammonium chloride, octyltrimethyl ammonium chloride, dodecyltrimethyl ammonium chloride, hexadecyltrimethyl ammonium chloride, octyldimethylbenzyl ammonium chloride, decyldimethylbenzyl ammonium chloride, stearyldimethylbenzyl ammonium chloride, didodecyldimethyl ammonium chloride, dioctadecyldimethyl ammonium chloride, tallowtrimethyl ammonium chloride, dihydrogenated tallow dimethyl ammonium chloride (e.g., Arquad 2HT/75 from Akzo Nobel), cocotrimethyl ammonium chloride, PEG-2-oleammonium chloride, the corresponding hydroxides thereof, or mixtures of two or more thereof. Other suitable cationic surfactants include, but are not limited to, those materials having the CTFA designations Quaternium-5, Quaternium-31 and Quaternium-18.

One cationic surfactant that is useful in personal care products (e.g., conditioners) in accordance with the present invention is cetyltrimethylammonium chloride which is available commercially from, for example, Hoechst Celanese as GENAMIN CTAC. Another cationic surfactant that is useful in personal care products (e.g., conditioners) in accordance with the present invention is behenyltrimethylammonium chloride which is available commercially from, for example, Clariant as GENAMIN KDMP.

Another example of a class of suitable cationic surfactants for use in the invention is a combination of compounds according to Formula (II) with an acid, where Formula (II) is an amidoamine:

R¹CONH(CH₂)_{b}N(R²)(R³) (II)

in which R¹ is a hydrocarbyl chain having 10 or more carbon atoms, R² and R³ are independently selected from hydrocarbyl chains of from 1 to 10 carbon atoms, and b is an integer from 1 to 10; and an acid.

As used herein, the term hydrocarbyl chain means an alkyl or alkenyl chain. In one embodiment, amidoamine compounds are those corresponding to Formula (I) in which R¹ is a hydrocarbyl residue having from 11 to 24 carbon atoms, R² and R³ are each independently hydrocarbyl residues, such as alkyl groups, having from 1 to 4 carbon atoms, and b is an integer from 1 to 4. In another embodiment, R² and R³ are methyl or ethyl groups. In still another embodiment, b is 2 or 3 (i.e., an ethylene or propylene group).

In one embodiment, amidoamines useful in conjunction with the presen invention include, but are not limited to, stearamido-propyldimethylamine, stearamido-propyldiethylamine, stearamido-ethyldiethylamine, stearamido-ethyldimethylamine, palmitamido-propyldimethylamine, palmitamido-propyldiethylamine, palmitamido-ethyldiethylamine, palmitamido-ethyldimethylamine, behenamido-propyldimethylamine, behenamido-propyldiethylmine, behenamido-ethyldiethylamine, behenamido-ethyldimethylamine, arachidamido-propyldimethylamine, arachidamido-propyldiethylamine, arachidamido-ethyldiethylamine, arachidamido-ethyldimethylamine, or mixtures of two or more thereof.

In still another embodiment, amidoamines useful in conjunction with the presen invention include, but are not limited to, stearamido-propyldimethylamine, stearamido-ethyldiethylamine, or mixtures thereof.

Commercially available amidoamines useful herein include, but are not limited to, stearamido-propyldimethylamine sold under the tradenames LEXAMINE^{®} S-13 (available from Inolex of Philadelphia Pa., USA) and AMIDOAMINE^{®} MSP (available from Nikko of Tokyo, Japan); stearamido-ethyldiethylamine sold under the tradename AMIDOAMINE^{®} S (available from Nikko of Tokyo, Japan); behenamide propyldimethylamine sold under the tradename INCROMINE^{®} BB (available from Croda of North Humberside, England); and various amidoamines sold under the tradename SCHERCODINE^{®} (available from Scher of Clifton N.J., USA).

The acid portion of the above-mentioned surfactant can be any organic or mineral acid which is capable of protonating the amidoamine in a personal care product (e.g., a hair treatment composition). Suitable acids for use in conjunction with the acid portion of the above-mentioned surfactant include, but are not limited to, hydrochloric acid, acetic acid, tartaric acid, fumaric acid, lactic acid, malic acid, succinic acid, or mixtures of two or more thereof. In one embodiment, the acid is selected from acetic acid, tartaric acid, hydrochloric acid, fumaric acid, or mixtures thereof.

The primary role of the acid is to protonate the amidoamine in the personal care product (e.g., a hair treatment composition) thus forming a tertiary amine salt (TAS) in situ in the personal care product. The TAS in effect becomes a non-permanent quaternary ammonium or pseudo-quaternary ammonium cationic surfactant. In one embodiment, the acid is included in a sufficient amount to protonate all the amidoamine present (i.e., at a level which is at least equimolar to the amount of amidoamine present in the composition). Mixtures of any of the above described cationic surfactants may also be used.

In two in one shampoo and conditioners and conditioning formulations of the kind covered within the scope of this invention, the level of cationic surfactant will generally range from 0.01 weight percent to 10 weight percent, or from 0.05 weight percent to 7.5 weight percent, or even from 0.1 weight percent to 5 weight percent, based on the total weight of the composition.

In another embodiment, the present invention utilizes a combination of fatty alcohols and cationic surfactants in conditioning compositions is believed to be advantageous, because this leads to the formation of a lamellar phase, in which the cationic surfactant is dispersed. Representative fatty alcohols comprise from 8 to 22 carbon atoms, or from 16 to 22. Fatty alcohols are typically compounds containing straight chain alkyl groups. Examples of suitable fatty alcohols include, but are not limited to, cetyl alcohol, stearyl alcohol, or mixtures of two or more thereof. The use of these materials is also advantageous in that they can contribute to the overall conditioning properties of compositions of the invention.

The level of fatty alcohol in the compositions of the present invention is in the range of from 0.01 weight percent to 10 weight percent, or from 0.1 weight percent to 8 weight percent, or from 0.2 weight percent to 7 weight percent, or even from 0.3 weight percent to 6 weight percent, based on the total weight of the composition.

The weight ratio of cationic surfactant to fatty alcohol is, in one embodiment, in the range of from 1:1 to 1:10, or from 1:1.5 to 1:8, or even from 1:2 to 1:5. In one instance, if the weight ratio of cationic surfactant to fatty alcohol is too high, this can lead to eye irritancy from the composition. In another instance, if the weight ratio of cationic surfactant to fatty alcohol is too low, it can make the hair feel squeaky for some users.

### Mixed Surfactants:

In still another embodiment, the blends of the present invention can include a mixture two or more different types of surfactants so long as at least one anionic surfactant, component (a), and at least one amphoteric or zwitterionic surfactant, component (b), are present as described above. In one embodiment, the blends of the present invention can utilize a mixture of two or more anionic surfactants with one or more amphoteric surfactants, or a mixture of one more anionic surfactants with two or more amphoteric surfactants. In one embodiment, the amount of the anionic surfactant(s) combined with the amphoteric surfactant(s) yield a total surfactant weight ranging in the range of from 4 weight percent active matter (AM) to 25 weight percent active matter, or from 6 weight percent AM to 20 weight percent AM. In one embodiment, the ratio of the total amount of the anionic surfactant(s) to the total amount of the amphoteric surfactant(s) is in the range of from 1:10 to 9:1, or from 1.25:1 to 8:1, or from 1.33:1 to 7:1, or from 1.5:1 to 6:1, or from 1.75:1 to 5:1, or even from 2:1 to 4:1. In the case of the above ratios, all of the ratios are based upon the active matter contents of the anionic surfactant(s) versus the active matter of the amphoteric surfactant(s). Active matter content is determined by taking the concentration value of any given material and multiplying that number with the amount (in this case the weight) of the material that is provided. For example, for 100 grams of a surfactant having an active concentration of 30% the active matter weight content would be 30 grams.

### Polymer Component (c):

As discussed above, in one embodiment the surfactant-polymer blends of the present invention comprises: (a) the at least one anionic surfactant; (b) the at least one amphoteric or zwitterionic surfactant; and (c) the at least one hydrophobically-modified polymer formed from a mixture comprising: (i) the α,β-ethylenically unsaturated carboxylic acid monomer; (ii) the nonionic, co-polymerizable α,β-ethylenically unsaturated monomer and (iii) the ethylenically unsaturated hydrophobically-modified associative monomer. In another embodiment, the surfactant-polymer blends of the present invention comprises: (a) the at least one anionic surfactant; (b) the at least one amphoteric or zwitterionic surfactant; and (c) the at least one hydrophobically-modified polymer formed from a mixture comprising: (i) the α,β-ethylenically unsaturated carboxylic acid monomer; (ii) the nonionic, co-polymerizable α,β-ethylenically unsaturated monomer, and (iii) the ethylenically unsaturated hydrophobically modified associative monomer; and (d) the at least one salt. The blends of this embodiment are referred to herein as surfactant-polymer-salt blends. The details of the polymer component of the present invention will now be discussed herein.

Thus, in one embodiment, the polymer component (c) of the present invention is a hydrophobically-modified alkali-soluble copolymer that contains hydrophobic substituents in the polymer backbone. In one embodiment, the polymer component (c) of the present invention is an aqueous emulsion copolymer of (i) the α,β-ethylenically unsaturated carboxylic acid monomer; (ii) the nonionic, co-polymerizable α,β-ethylenically unsaturated monomer; and (iii) the ethylenically unsaturated hydrophobically-modified associative monomer.

Turning to component (i) that is used to form the at least one hydrophobically-modified polymer.

The component (i) acid monomers are selected from acrylic, methacrylic, crotonic, acyloxypropionic, maleic, fumaric, itaconic, aconitic, half- or mono-esters of dibasic or tribasic acids such as the monobutyl ester of maleic acid, or suitable mixtures of two or more thereof. In another embodiment, acrylic acid, methacrylic acid, or a mixture thereof is used as the acid monomer of component (i).

Turning to component (ii) that is used to form the at least one component (ii) nonionic monomers are selected from C₁ to C₄ alkyl acrylates and methacrylates such as methyl acrylate, ethyl acrylate, butyl acrylate, ethyl methacrylate, or suitable mixtures of two or more thereof.

Turning to component (iii) that is used to form the at least one hydrophobically-modfied polymer, component (iii) (the ethylenically unsaturated hydrophobically-modified associative monomer) that are utilized to form component (c) of the present invention is selected from cetyl polyethoxylated (meth)acrylate (CEM), cetearyl polyethoxylated (meth)acrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, lacceryl polyethoxylated (meth)acrylate, nonylphenol polyethoxylated (meth)acrylate (NEM), tristyrylphenol polyethoxylated (meth)acrylate (TEM), palmyl polyethoxylated itaconate (PEI), stearyl polyethoxylated itaconate (SEI), behenyl polyethoxylated itaconate (BEI), palmyl polyethoxylated maleate (PEML), stearyl polyethoxylated maleate (SEM), behenyl polyethoxylated maleate (BEML), palmyl polyethoxylated allyl ether (PEAE), stearyl polyethoxylated allyl ether (SEAE), behenyl polyethoxylated allyl ether (BEAE), where the polyethoxylated portion of the foregoing monomers contain from 3 to 200 ethylene oxide repeating units, from 5 to 100 ethylene oxide repeating units, or even 15 to 60 ethylene oxide repeating units.

In one embodiment, the amounts of components (i), (ii) and (iii) that are utilized in a suitable polymerization reaction to produce the at least one hydrophobically-modified polymer of component (c) of the blends of the present invention are as follows: (i) from 2.5 weight percent to 75 weight percent of the one or more α,β ethylenically unsaturated carboxylic acid monomers; (ii) from 0 weight percent to 90 weight percent of the one or more nonionic, co-polymerizable α,β-ethylenically unsaturated monomers; and (iii) from 0.5 weight percent to 40 weight percent of the one or more ethylenically unsaturated hydrophobically-modified associative monomers. In another embodiment, the amounts of components (i), (ii) and (iii) are as follows: (i) from 5 weight percent to 70 weight percent; (ii) from 5 weight percent to 85 weight percent; and (iii) from 1 weight percent to 30 weight percent. In still another embodiment, the amounts of components (i), (ii) and (iii) are as follows: (i) from 10 weight percent to 60 weight percent; (ii) from 10 weight percent to 80 weight percent; and (iii) from 2.5 weight percent to 25 weight percent. In still yet another embodiment, the amounts of components (i), (ii) and (iii) are as follows: (i) from 20 weight percent to 55 weight percent; (ii) from 20 weight percent to 75 weight percent; and (iii) from 5 weight percent to 20 weight percent. In still yet another embodiment, the amounts of components (i), (ii) and (iii) are as follows: (i) from 30 weight percent to 50 weight percent; (ii) from 30 weight percent to 70 weight percent; and (iii) from 7.5 weight percent to 15 weight percent. Although the total amount of each monomer or component of a various "charge" may individually total more then 100 weight percent when each component is taken individually and totaled using the broadest amounts disclosed herein, one of skill in the art will realize that this is not the case.

In another embodiment, polymer component (c) of the blends of the present invention can be crosslinked or non-crosslinked (i.e., linear). In another embodiment, polymer component (c) of the blends of the present invention is non-crosslinked (i.e., linear). In one embodiment, component (c) of the blends of the present invention can be prepared by conventional emulsion polymerization techniques using appropriate emulsifiers for emulsifying the monomers and maintaining the polymer obtained in a stable-dispersed condition. In this instance, polymer component (c) comprises an aqueous emulsion comprising from 1 weight percent to 60 weight percent of one or more of the above polymer components with the remainder of the emulsion being primarily water.

One suitable preparation technique is described in United States Patent No. 4,616,074. In the process disclosed in United States Patent No. 4,616,074, the polymerization is carried out in continuous, semi-continuous or batch fashion with the polymerization reaction initiated at 40°C to 90°C. A thermal decomposition initiator such as ammonium persulfate or potassium persulfate is used, or at lower temperatures a redox initiator such as t-butyl hydroperoxide/bisulfite is used. An anionic emulsifier is normally included in the reaction medium at a concentration of 1 weight percent to 3 weight percent, based on the total weight of the monomer charge, so as to maintain a stable aqueous dispersion. Suitable emulsifiers include, but are not limited to, sodium lauryl sulfate, sodium dodecylbenzene sulfonate, as well as other ammonium and alkali metal alkyl aryl sulfonates and alkyl sulfates. The polymerization is carried out at a pH of less than 5.0 to maintain the insolubility of the copolymer in the continuous water phase. The polymer dispersions have relatively low viscosity even at solids content of from 20 weight percent to 40 weight percent or higher.

Upon addition of an alkali to neutralize at least a portion of the free carboxyl groups, aqueous systems containing one or more polymer components (b) of the present invention markedly thicken. Any alkali can be used to neutralize the copolymers and can be an alkali metal hydroxide such as sodium or potassium hydroxide, sodium carbonate, or other bases such as ammonium hydroxide, methylamine, diethylamine, triethylamine and triethanolamine. In one embodiment, an effective neutralizing amount of alkali is used and depending on the particular application, it is an amount sufficient to adjust the pH of the mixture to above 6.5. In another embodiment, the amount of neutralizing agent used is an amount sufficient to adjust the pH of the mixture to from 6.5 to 14, or from 7 to 12, or from 7.5 to 10, or even from 7.0 to 7.5. Here, as well as elsewhere in the specification and claims, individual numerical values, or limits, can be combined to form additional non-disclosed and/or non-stated ranges.

### Salt Component (d):

In the blends of the present invention, salt component (d) is at least one salt selected from inorganic monovalent electrolytes selected from lithium chloride, sodium chloride, ammonium chloride, potassium chloride, or mixtures of any two or more thereof. In another embodiment, one or more organic salts are utilized as component (d). These organic salts are selected from sodium benzoate, sodium salicylate, or a mixture of any two or more thereof. In still yet another embodiment, any combination of two or more of any of the above salt compounds can be utilized as component (d) of the present invention.

### Optional Components.

In still another embodiment, the blends of the present invention can include one or more optional components that are typically found in personal care products, health care products, household care products, institutional and industrial care products, and industrial applications. Such optional components include, but are not limited to, one or more cationic polymers, one or more multipurpose polymers, one or more compounds that enhancing performance and/or consumer acceptability, or mixtures of two or more thereof. Such one or more compounds that enhancing performance and/or consumer acceptability include fragrance, dyes and pigments, pH adjusting agents, pearlescers or opacifiers, other viscosity modifiers, preservatives, and natural hair nutrients such as botanicals, fruit extracts, sugar derivatives, solvents and amino acids.

### Cationic Polymers:

In one embodiment, the optional cationic polymers that can be utilized in conjunction with the blends of the present invention can be homopolymers which are cationically substituted or can be formed from two or more types of monomers. The weight average (M_{w}) molecular weight of the polymers are in the range of 75,000 daltons to 2,500,000 daltons, or even from 100,000 daltons to 2,000,000 daltons.

In one embodiment, the optional cationic polymers that can be utilized in conjunction with the blends of the present invention have cationic nitrogen-containing groups such as quaternary ammonium or protonated amino groups, or a mixture thereof. In one personal care embodiment, if the molecular weight of the polymer is too low, then the conditioning effect is poor. If too high, then there can be problems of high extensional viscosity leading to stringiness of the composition when it is poured.

In one embodiment, the cationic nitrogen-containing group will generally be present as a substituent on a fraction of the total monomer units of the cationic polymer. Thus, in embodiments where the cationic polymer is not a homolymer it can contain spacer non-cationic monomer units. Such polymers are described in the CTFA Cosmetic Ingredient Directory, 3rd Edition. In one embodiment, the ratio of the cationic to non-cationic monomer units is selected to give polymers having a cationic charge density in the required range, which in one embodiment is from 0.2 to 3.0 meq/gm. The cationic charge density of the polymer is suitably determined via the Kjeldahl method as described in the US Pharmacopoeia under chemical tests for nitrogen determination.

In one embodiment, suitable cationic polymers include, but are not limited to, copolymers of vinyl monomers having cationic amine or quaternary ammonium functionalities with water soluble spacer monomers such as (meth)acrylamide, alkyl and dialkyl(meth)acrylamides, alkyl(meth)acrylate, vinyl caprolactone and vinyl pyrrolidine. The alkyl and dialkyl substituted monomers can contain C₁ to C₇ alkyl groups, or even C₁ to C₃ alkyl groups. Other suitable spacers include vinyl esters, vinyl alcohol, maleic anhydride, propylene glycol and ethylene glycol.

In one embodiment, the cationic amines can be primary, secondary or tertiary amines, depending upon the particular species and the pH of the composition. In another embodiment, secondary and tertiary amines, or even tertiary amines are utilized. Amine substituted vinyl monomers and amines can be polymerized in the amine form and then converted to ammonium by quaternization. In still another embodiment, the cationic polymers can comprise mixtures of monomer units derived from amine- and/or quaternary ammonium-substituted monomer and/or compatible spacer monomers.

Suitable cationic polymers include, but are not limited to, cationic diallyl quaternary ammonium-containing polymers including, but not limited to, dimethyldiallyl ammonium chloride homopolymer and copolymers of acrylamide and dimethyldiallyl ammonium chloride, referred to in the industry (CTFA) as Polyquaternium 6 and Polyquaternium 7, respectively; mineral acid salts of amino-alkyl esters of homo- and co-polymers of unsaturated carboxylic acids having from 3 to 5 carbon atoms (as described in United States Patent No. 4,000,256); cationic polyacrylamides (as described in WO 95/22311) or suitable mixtures of two or more thereof.

Other cationic polymers that can be used in conjunction with the blends of the present invention include, but are not limited to, cationic polysaccharide polymers, such as cationic cellulose derivatives, cationic starch derivatives, and cationic guar gum derivatives. Cationic polysaccharide polymers suitable for use in compositions of the invention include, but are not limited to, monomers having the Formula shown below:

E-O-[R₁₀-N⁺(R₁₁)(R₁₂)(R₁₃)X₃]

where E is an anhydroglucose residual group, such as a starch or cellulose anhydroglucose residual; R₁₀ is an alkylene, oxyalkylene, polyoxyalkylene, or hydroxyalkylene group, or combination thereof; R₁₁, R₁₂, and R₁₃ are independently selected from alkyl, aryl, alkylaryl, arylalkyl, alkoxyalkyl, or alkoxyaryl groups, where each group can independently contain from up to 18 carbon atoms. The total number of carbon atoms for each cationic moiety (i.e., the sum of carbon atoms in R₁₁, R₁₂ and R₁₃) is 54 or less, 40 or less, 30 or less, or even 20 or less; and X₃ is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation under, for example, the tradename Polymer LM-200. Other suitable cationic polysaccharide polymers include quaternary nitrogen-containing cellulose ethers (e.g., those described in United States Patent No. 3,962,418), and copolymers of etherified cellulose and starch (e.g., those described in United States Patent No. 3,950,581), or mixtures of two or more thereof.

A particularly suitable type of cationic polysaccharide polymer that can be used is a cationic guar gum derivative, such as guar hydroxypropyltrimethyl ammonium chloride (commercially available from Rhodia in their JAGUAR^{®} series). Examples of such materials include, but are not limited to, JAGUAR^{®} C13S, JAGUAR^{®} C14, JAGUAR^{®} C15, JAGUAR^{®} C17, JAGUAR^{®} C16, JAGUAR^{®} CHT, and JAGUAR^{®} C162.

In still another embodiment, mixtures of any of the above cationic polymers can be used. If present, the one or more cationic polymers are present in a personal care composition (e.g., a shampoo composition) at levels of from 0.01 weight percent to about 5 weight percent, or from 0.05 weight percent to 1 weight percent, or even from 0.08 weight percent to 0.5 weight percent, based on the total weight of the composition.

### Applications for the Surfactant-Polymer Blends of the Present Invention:

As used herein the term "blend" or "blends" refers to the surfactant-polymer blends of the present invention regardless of whether or not such blends contain one or more salts compounds disclosed herein. As such, the terms "blend," "blends," "surfactant-polymer blend," or "surfactant-polymer blends" are meant to encompass all of the embodiments of the present invention as disclosed herein.

The surfactant-polymer blends compositions of the invention can be utilized in a variety of products for personal care, health care, home care, institutional and industrial (collectively "I&I) care, and in a variety of products for medical and industrial applications. The inventive blends of the present invention can be employed as emulsifiers, stabilizers, suspending agents, deposition aids for enhancing the efficacy, deposition or delivery of chemically and physiologically active ingredients and cosmetic materials, film formers, thickeners, rheology modifiers, hair fixatives, conditioners, moisturizers, spreading aids, carriers, and as an agent for improving the psychosensory, and aesthetic properties of a formulation in which they are included.

### Personal Care and Health Care Applications:

The surfactant-polymer blends of the present invention are, in one embodiment, suitable for the preparation of personal care (e.g., cosmetics, toiletries, cosmeceuticals), topical health care products and medical products including, but not limited to, hair care products, such as shampoos (including combination shampoos, such as "two-in-one" conditioning shampoos and anti-dandruff shampoos); post-shampoo rinses; setting and style maintenance agents including setting aids and fixatives, such as gels and sprays, grooming aids, such as pomades, conditioners, perms, relaxers, hair smoothing products, and the like; skin care products (e.g., facial, body, hands, scalp and feet), such as creams, lotions, conditioners, deodorants, antiperspirants and cleansing products; anti-acne products; anti-aging products (e.g., exfoliant, keratolytic, anti-cellulite, anti-wrinkle, and the like); skin protectants such as sunscreens, sunblock, barrier creams, oils, silicones, and the like; skin color products (e.g., whiteners, lighteners, sunless tanning accelerators, and the like); hair colorants (e.g., hair dyes, hair color rinses, highlighters, bleaches and the like); pigmented skin colorants (e.g., face and body make-ups, foundation creams, mascara, rouge, lip products, and the like); bath and shower products (e.g., body cleansers, body washes, shower gels, liquid soaps, soap bars, syndet bars, conditioning liquid bath oil, bubble bath, bath powders, therapeutic cleaners, acne control products, facial cleansers and the like); nail care products (e.g., polishes, polish removers, strengtheners, lengtheners, hardeners, cuticle removers, softeners, and the like); and any aqueous composition to which an effective amount of surfactant-polymer blend composition can be incorporated for achieving a beneficial or desirable, physical or chemical effect therein during storage and/or usage.

Toiletries and health and beauty aids, commonly referred to as HBAs, containing a surfactant-polymer blend of the invention, can include, but are not limited to, hair-removal products (e.g., shaving creams and lotions, depilatories, after-shave skin conditioners, and the like); deodorants and antiperspirants; oral care products (e.g., for the mouth, teeth and gums), such as mouthwash, dentifrice, such as toothpaste, tooth powder, tooth polishes, tooth whiteners, breath fresheners, denture adhesives, and the like; facial and body hair bleach; and the like. Other health and beauty aids that can contain surfactant-polymer blends of the present invention include, but are not limited to, sunless tanning applications containing artificial tanning accelerators, such as dihydroxyacetone (DHA), tyrosine, tyrosine esters, and the like; skin de-pigmenting, whitening, and lightening formulations containing such active ingredients as kojic acid, hydroquinone, arbutin, fruital, vegetal or plant extracts, (lemon peel extract, chamomile, green tea, paper mulberry extract, and the like), ascorbyl acid derivatives (ascorbyl palmitate, ascorbyl stearate, magnesium ascorbyl phosphate, and the like); foot care products, such as keratolytic corn and callous removers, foot soaks, foot powders (medicated, such as antifungal athlete's foot powder, ointments, sprays, and the like, and antiperspirant powders, or non-medicated moisture absorbent powder), liquid foot sprays (non-medicated, such as cooling, and deodorant sprays, and medicated antifungal sprays, antiperspirant sprays, and the like), and foot and toenail conditioners (lotions and creams, nail softeners, and the like).

Topical health and beauty aids that can include the surfactant-polymer blends of the invention (e.g., thickeners, rheology modifiers, spreading aids, deposition aids and film formers) include, but are not limited to, skin protective spray, cream, lotion, gel, stick and powder products, such as insect repellants, itch relief, antiseptics, disinfectants, sun blocks, sun screens, skin tightening and toning milks and lotions, wart removal compositions, and the like.

Other health care products in which surfactant-polymer blends can be included are medical products, such as topical and non-topical pharmaceuticals, and devices. In the formulation of pharmaceuticals, a surfactant-polymer blend of the invention can be employed as a thickener or rheology modifier in such products as creams, pomades, gels, pastes, ointments, tablets, gel capsules, purgative fluids (e.g., enemas, emetics, colonics, and the like), suppositories, anti-fungal foams, eye products (e.g., ophthalmic products, such as eye-drops, artificial tears, glaucoma drug delivery drops, contact lens cleaner, and the like), ear products (e.g., wax softeners, wax removers, otitis drug delivery drops, and the like), nasal products (e.g., drops, ointments, sprays, and the like), and wound care (e.g., liquid bandages, wound dressings, antibiotic creams, ointments, and the like), without limitation thereto.

The film-forming character of compositions that utilize the surfactant-polymer blends of the present invention make compositions containing a blend of the present invention particularly suitable as a vehicle for topical medical compositions for promoting and enhancing the transdermal delivery of active ingredients to or through the skin, for enhancing the efficacy of anti-acne agents formulations and topical analgesics, and for controlling release of drugs, such as antacids from tablets, or syrups; controlling drug release from tablets, lozenges, chewables, and the like in the mildly acidic environment of the mouth; or from suppositories, ointments, creams, and the like in the mildly acidic environment of the vagina; to promote deposition of dandruff control agents from shampoos, salves, and the like; to enhance the deposition of colorants on skin from pigmented cosmetics (e.g., make-ups, lipsticks, rouges, and the like) and on hair from hair dyes, and the like.

The surfactant-polymer blends of the present invention can be employed, without being limited thereto, as a lubricant coating for medical devices, such as soft tissue implants, surgical gloves, catheters, cannulae, and the like, as removable protective film coatings for medical instruments, wound dressings, and the like, as a muco-adhesive, especially in the acid environment of the stomach, as a carrier and thickener in formulated products for medical applications, such as disinfectant hand creams, antiviral products, antibiotic ointments, sprays and creams, non-drip, sprayable disinfectant in hospitals, hard surface antimicrobial finish applied during routine maintenance, and the like.

### Home Care and Institutional and Industrial Applications:

The surfactant-polymer blends of the present invention can be used in home care, and I&I applications, for example, as a thickener or rheology modifier, to improve formulation efficiency through "cling-on-surface" or for improving the efficacy of disinfectants, detersive surfactants, and biocidal formulations, and to synergistically improve fabric softening efficacy in combination with traditional fabric softeners. Typical household and I&I products that may contain polymers of the invention include, but are not limited to, laundry and fabric care products, such as detergents, laundry pre-spotting removal products, fabric softeners (liquids or sheets), ironing sprays, dry cleaning aids, anti-wrinkle sprays, spot removers and the like; hard surface cleansers for the kitchen and bathroom and utilities and appliances employed or located therein, such as toilet bowl gels, tub and shower cleaners, hard water deposit removers, floor and tile cleansers, wall cleansers, floor and chrome fixture polishes, alkali-strippable vinyl floor cleaners, marble and ceramic cleaners, air freshener gels, liquid cleansers for dishes, and the like; disinfectant cleaners, such as toilet bowl and bidet cleaners, disinfectant hand soaps, room deodorizers, and the like.

### Institutional and Industrial Applications:

The surfactant-polymer blends of the present invention can be utilized as thickeners, rheology modifiers, dispersants, stabilizers, promoters, and the like, in institutional and industrial product applications, such as, without being limited thereto, textiles (e.g., processing, finishing, printing, and dyeing aids, protective washable surface coatings, manufacture of synthetic leather by saturation of non-woven fabrics, and the like, manufacturing of woven fabrics, non-woven fabrics, natural and synthetic fibers and the like); water treatments (e.g., waste water, cooling water, potable water purification, and the like); chemical spill containments (e.g., acid-spill absorbent, and the like); leather and hide processing (e.g., processing aids, finishing, coating, embossing, and the like); paper and papermaking (e.g., surface coatings, such as pigmented coatings, antistatic coatings, and the like, pulp binders, surface sizings, dry and wet strength enhancers, manufacture of wet-laid felts, and the like); printing (e.g., inks, anti-wicking ink-jet printer inks, thickeners for ink formulations containing cationic dyes for printing acrylic fabrics, and the like); paints (e.g., pigment and grinding additive, crosslinking agent for epoxy latex emulsions, particulate-suspending aid for clays, pigments, and the like); industrial plant effluent treatment (e.g., flocculants for phenolics in paper mill effluent, and the like); metal working (e.g., acid etch cleaners, low pH metal coatings, pickling agents in cold rolled steel processing, and the like); adhesives (e.g., clear adhesives, adhesion promoters for metal, plastic, wood, and the like, non-woven floc adhesive tie coatings, bonding, and the like); wood preservation; and industrial construction products for buildings and roads (e.g., cement plasticizers, asphalt emulsion stabilizers at low pH, acid etch for cement, consistency modifiers of concrete, mortar, putty, and the like). The surfactant-polymer blends of the present invention are particularly useful as thickeners for rust removers, acid truck cleaners, scale removers, and the like.

The surfactant-polymer blends of the present invention are particularly useful, in one embodiment, as emulsification aids for water-insoluble (hydrophobic) oily materials such as natural and synthetic oils, fats, and waxes, including, for example, vegetable oils, animal oils and fats, paraffin oils and waxes, silicone oils and waxes; and the like. Many oily materials are used as solvents, carriers, emollients, or conditioning agents, for example, in hair and skin care products.

The surfactant-polymer blends of the present invention are surprisingly useful stabilizers of water insoluble materials such as silicone fluids, rigid silicones, amino silicones, and dimethicone copolyols. Silicone fluids which are commonly used in shampoo products, such as the so-called "two-in-one" combination cleansing/conditioning shampoos. The polymers of the present invention are surprisingly effective for stabilizing and depositing two-in-one type shampoo formulations containing suitable silicone products of low, medium and high molecular weight silicone polyether fluids (193C, 5330) and resins; low, medium, high viscosity dimethyl silicone fluids/gums/resins or in the form of nonionic small and large particle size emulsions (MEM 1664, MEM 1310, 5-7137, 2-1352, MEM 1784, MEM 1310, MEM 1491, 5-7137, and MEM 2220), and anionic emulsions (MEM 1784); aminosilicone fluids with low and high amine content (8500, 2-8566, AP-8087); amino glycol copolymer; amino phenyl resin; low and high viscosity cationic emulsions (949, 2-8194), nonionic microemulsions; silicone quaternary microemulsions (5-7113); phenyl silicone (556) fluids, silicone wax (AMS C-30), silicone elastomer blend (9040) and the like, and mixtures of two or more thereof.

The surfactant-polymer blends of the present invention are particularly useful, in one embodiment, as suspending agents for particulates, such as mica, pearlizing agents, beads, and the like, making them suitable for dermal products containing particulates, micro-abrasives, and abrasives, such as bath and shower gels, masks and skin cleansers containing exfoliative scrub agents. Numerous cosmetically useful particulate exfoliating agents are known in the art, and the selection and amount is determined by the exfoliating effect desired from the use of the composition, as recognized by those skilled in the cosmetic arts.

Suitable exfoliating agents include, but are not limited to, biological abrasives, inorganic abrasives, synthetic polymers, and the like, and mixtures thereof. Biological abrasives include, but are not limited to, shell, seed, and kernel or stone granules or powders, obtained from nuts, such as from walnut (Juglans regia) shells, almonds, pecans, and the like; fruital sources, such as apricots, avocados, coconuts, olives, peaches, and the like; vegetal sources, such as corn cob, oat bran, rice, rose hip seed, jojoba (wax, seed powder), microcrystalline cellulose, ground loofa, ground seaweed, and the like; animal sources, such as oyster shell, silk, microcrystalline collagen, and the like. Inorganic abrasives include, but are not limited to, stannic oxide, talc, silica (hydrated, colloidal and the like), kaolin, precipitated chalk, salts (sodium chloride, dead sea salt, and the like), ground pumice, and the like. Synthetic polymers include, but are not limited to, microcrystalline polyamides (nylons), microcrystalline polyesters (polycarbonates), polymethyl (meth)acrylate microbeads (PMMA microbeads) and the like. The polymers of the present invention are also useful for suspending gaseous bubbles in a liquid medium.

The surfactant-polymer blends are useful as thickeners, rheology modifiers, deposition aids, and film-formers in a variety of dermatological, pharmaceutical, and cosmeceutical compositions employed for topically ameliorating skin and scalp conditions caused by perspiration, inflammation, swelling, drying, dandruff, photo-damage, aging, acne, and the like, containing pharmaceutical and cosmeceutical active ingredients, conditioners, surfactants, moisturizers, antioxidants, exfoliants (described above), keratolytic agents, botanicals, vitamins, and the like, and combinations thereof.

Suitable examples of pharmaceutical and cosmeceutical active ingredients include, but are not limited to, caffeine, vitamin C, vitamin D, vitamin E, pantothenic acid (vitamine B5), anti-stretch mark compounds, astringents (e.g., alum, oatmeal, yarrow, witch hazel, bayberry, and isopropyl alcohol), draining compounds, hair growth compounds (e.g., monoxidil), skin and hair nourishing compounds, skin and hair protecting compounds, self-tanning compounds (e.g., mono- or polycarbonyl compounds such as, for example, isatin, alloxan, ninhydrin, glyceraldehyde, mesotartaric aldehyde, glutaraldehyde, erythrulose, tyrosine, tyrosine esters, and dihydroxyacetone), sunscreens (e.g., ethylhexyl methoxy cinnamate, octinoxate, octisalate, oxybenzone), skin lighteners (e.g., kojic acid, hydroquinone, arbutin, fruital, vegetal or plant extracts, such as lemon peel extract, chamomile, green tea, paper mulberry extract, and the like, ascorbyl acid derivatives, such as ascorbyl palmitate, ascorbyl stearate, magnesium ascorbyl phosphate, and the like), lip plumping compounds, anti-aging, anti-cellulite, and anti-acne compounds (e.g., acidic agents such as alpha-hydroxy acids (AHAs), beta-hydroxy acids (BHAs), alpha amino-acids, alpha-keto acids (AKAs), acetic acid, azelaic acid, and mixtures thereof), anti-dandruff compounds (e.g., zinc pyrithione, zinc omadine, miconazole nitrate, selenium sulfide, piroctone olamine) anti-inflammatory compounds (e.g., aspirin, ibuprofen, and naproxen), analgesics (e.g., acetaminophen), antioxidant compounds, antiperspirant compounds, deodorant compounds (e.g., 2-amino-2-methyl-1-propanol (AMP), ammonium phenolsulfonate; benzalkonium chloride; benzethonium chloride, bromochlorophene, cetyltrimethylammonium bromide, cetyl pyridinium chloride, chlorophyllin-copper complex, chlorothymol, chloroxylenol, cloflucarban, dequalinium chloride, dichlorophene, dichloro-m-xylenol, disodium dihydroxyethyl sulfosuccinylundecylenate, domiphen bromide, hexachlorophene, lauryl pyridinium chloride, methylbenzethonium chloride, phenol, sodium bicarbonate, sodium phenolsulfonate, triclocarban, triclosan, zinc phenolsulfonate, zinc ricinoleate, and mixtures thereof), hair fixative polymers (e.g., natural and synthetic polymers such as, for example, polyacrylates, polyvinyls, polyesters, polyurethanes, polyamides, modified cellulose, starches, and mixtures thereof), hair and skin conditioners (e.g., synthetic oils, natural oils, such as vegetable, plant and animal oils, mineral oils, natural and synthetic waxes, cationic polymers, monomeric and polymeric quaternized ammonium salt compounds, silicones such as silicone oils, resins and gums, proteins, hydrolyzed proteins, fatty acids, fatty amines; and mixtures thereof); and suitable mixtures of two or more of the above.

In one cosmeceutical aspect, a surfactant-polymer blend can be employed as a thickener, rheology modifier and/or a deposition aid for active skin treatment lotions and creams containing, as active ingredients, acidic anti-aging, anti-cellulite, and anti-acne agents, hydroxy carboxylic acids, such as alpha-hydroxy acid (AHA), beta-hydroxy acid (BHA), alpha-amino acid, alpha-keto acids (AKAs), and mixtures thereof. Suitable AHAs include, but are not limited to, lactic acid, glycolic acid, fruit acids, such as malic acid, citric acid, tartaric acid, extracts of natural compounds containing AHA, such as apple extract, apricot extract, and the like, honey extract, 2-hydroxyoctanoic acid, glyceric acid (dihydroxypropionic acid), tartronic acid (hydroxypropanedioic acid), gluconic acid, mandelic acid, benzilic acid, alpha-lopioc acid, AHA salts and derivatives, such as arginine glycolate, ammonium lactate, sodium lactate, alpha-hydroxybutyric acid, alpha-hydroxyisobutyric acid, alpha-hydroxyisocaproic acid, alpha-hydroxyisovaleric acid, atrolactic acid, and the like. Suitable BHAs include, but are not limited to, 3-hydroxy propanoic acid, beta-hydroxybutyric acid, beta-phenyl lactic acid, beta-phenylpyruvic acid, salicylic acid, and the like. Suitable alpha-amino acids include, but are not limited to, alpha-amino dicarboxylic acids, such as aspartic acid, glutamic acid, and the like. Representative alpha-keto acids are pyruvic acid, acetopyruvic acid, and the like. Other active acidic agents include retinoic acid and its derivatives, halocarboxylic acids (e.g., trichloroacetic acid), acidic antioxidants (e.g., vitamin C), mineral acids, phytic ecid, lysophosphatidic acid, salicylic acid, derivatives of salicylic acid (e.g., 5-octanoylsalicylic acid), and the like. Typically the active acidic ingredient has a pH in the range of 0.5 to 5.

A discussion of the use and formulation of active skin treatment compositions is in COSMETICS & TOILETRIES^{®}, C&T Ingredient Resource Series, AHAs & Cellulite Products How They Work, published 1995, and Cosmeceuticals, published 1998, both available from Allured Publishing Corporation, incorporated herein by reference. Compositions containing alpha-amino acids acidified with ascorbic acid are described in United States Patent No. 6,197,317 and United States Patent Application Publication No. 2009/0029928, and a commercial cosmeceutical preparation utilizing these acids in an anti-aging, skin care regimen is sold under the tradename, AFAs, by exCel Cosmeceuticals (Bloomfield Hills, Michigan). The term "AFA," as described in the supplier's trade literature, was coined by the developer to describe the amino acid/vitamin C combination as Amino Fruit Acids and as the acronym for "Amino acid Filaggrin based Antioxidants." In addition to ingredients discussed above, other ingredients commonly used for anti-acne products, facial and body hair bleaches, and anti-septic products include oxidizing agents, such as hydrogen peroxide, benzoyl peroxide, and water-soluble inorganic persulfate compounds such as ammonium persulfate, potassium persulfate, and sodium persulfate.

Suitable antiperspirant agents that can be utilized according to the foregoing antiperspirant embodiment include conventional antiperspirant metal salts and complexes of metal salts. In one embodiment of the invention, the metal salts and metal salt complexes utilized as the antiperspirant agents are acidic and are based on aluminum and zirconium and combinations thereof. These salts include, but are not limited to, aluminum halides, aluminum hydroxyhalides, aluminum sulfate, zirconium (zirconyl) oxyhalides, zirconium (zirconyl)hydroxyhalides, and mixtures or complexes thereof. Complexes of aluminum and zirconium salts include, but are not limited to, aluminum and zirconium salt complexes with amino acids, such as, for example, glycine or complexes with a glycol, such as, for example, propylene glycol (PG) or polyethylene glycol (PEG). Exemplary antiperspirant agents include, but are not limited to, aluminum chloride, aluminum chlorohydrate, aluminum dichlorohydrate, aluminum sesquichlorohydrate, zirconyl hydroxychloride, aluminum chlorohydrex PEG (aluminum chlorohydrex polyethylene glycol), aluminum chlorohydrex PG (aluminum chlorohydrex propylene glycol), aluminum dichlorohydrex PEG (aluminum dichlorohydrex polyethylene glycol), aluminum dichlorohydrex PG (aluminum dichlorohydrex propylene glycol), aluminum sesquichlorohydrex PEG (aluminum sesquichlorohydrex polyethylene glycol), aluminum sesquichlorohydrex PG (aluminum sesquichlorohydrex propylene glycol), aluminum zirconium trichlorohyrate, aluminum zirconium tetrachlorohyrate, aluminum zirconium pentachlorohyrate, aluminum zirconium octachlorohyrate, aluminum zirconium chlorohydrex GLY (aluminum zirconium chlorohydrex glycine), aluminum zirconium trichlorohydrex GLY (aluminum zirconium trichlorohydrex glycine), aluminum zirconium tetrachlorohyrex GLY (aluminum zirconium tetrachlorohyrex glycine), aluminum zirconium pentachlorohyrex GLY (aluminum zirconium pentachlorohyrex glycine), and aluminum zirconium octachlorohyrex GLY (aluminum zirconium octachlorohyrex glycine). Other antiperspirant agents include, but are not limited to, ferric chloride and zirconium powder. Mixtures of any of the foregoing antiperspirant agents are also suitable for use in the present invention.

The amount of the antiperspirant agent incorporated into the antiperspirant compositions of the present invention is an amount that is sufficient to reduce the flow of perspiration from the location to which the antiperspirant product is applied, for example to the auxiliary area of the human body, while providing a suitably low pH to neutralize the cationic hydrophobically modified polymer to attain a desired viscosity. If the desired amount of antiperspirant agent loading is reached before the cationic hydrophobically modified polymer is sufficiently neutralized to achieve the desired viscosity profile, auxiliary acidification agents can be added to effect the desired viscosity profile.

Generally, the level of antiperspirant agent utilized in the compositions of the present invention range from 0.5 weight percent to 35 weight percent based on the total weight of the antiperspirant composition. In another embodiment, the amount of antiperspirant agent in the composition can range from 1 weight percent to 25 weight percent, or from 5 weight percent to 20 weight percent, or even from 10 weight percent to 15 weight percent, based on the total weight of the composition. The foregoing weight percentages are calculated on an anhydrous metal salt basis exclusive of the complexing agent (e.g., glycine or glycol). Here, as well as else where in the specification and claims, individual numerical values (including carbon atom numerical values), or limits, can be combined to form additional non-disclosed and/or non-stated ranges. Unless otherwise stated herein, the active ingredients can be present in effective amounts to accomplish their function, and are generally included individually at a level of from 0 weight percent to 35 weight percent, based on the weight of the total composition in which they are employed.

In one aspect the surfactant-polymer blends of the present invention can be used as a thickener, rheology modifier, film former, deposition aid, or as a dye or pigment suspending agent for promoting deposition of colorants on hair and skin. Colorants for hair can be temporary, semi-permanent or permanent hair dyes or color restorers that pigment the hair gradually. Temporary and semi-permanent hair dyes typically are rinses, gels, sprays, shampoos, sticks, and the like, and hair color restorers are typically in the form of hair dressings or emulsions. Permanent hair dyes, and longer-lasting semi-permanent hair dyes, are generally two-part products, one part containing the oxidative dye intermediates and dye couplers, and the other part containing stabilized oxidizing agent, usually hydrogen peroxide at pH 3 to 4, and are mixed together immediately before use. It is known that such two-part hair dyeing products are formulated with combinations of surfactant ingredients, usually nonionic surfactants or anionic surfactants, to thicken when the dye mixture is prepared. In addition to the foregoing literature, a general discussion of hair dyeing chemistry and compositions is in Brown et al, SCC Monograph, Permanent Hair Dyes, Society of Cosmetic Chemists (1996), incorporated herein by reference. The polymers of the present invention may be incorporated in one or both of the two-parts of such hair dyeing systems, either as the thickener for the acidic stabilized oxidizing portion or in the non-oxidizing portion to be thickened upon mixing with the acidic portion.

In another hair care embodiment, the inventive surfactant-polymer blends can be utilized in an amount effective to provide to the hair care composition a property, such as a hair fixative property, a hair conditioning property, a viscid property (thickening or rheology modifying), or a combination thereof. Optionally, the hair care composition can include one or more auxiliary film-forming agents, auxiliary hair fixative agent, auxiliary hair conditioning agent, auxiliary rheology modifying agent, or mixtures thereof.

The term "fixative" encompasses the properties of film-formation, adhesion, or a coating deposited on a surface on which the polymer is applied. The terms "hair setting," "hair styling" and "hair fixative" as commonly understood in the hair care arts, and as used herein, refer collectively to hair setting agents that are hair fixatives and film formers and which are topically applied to the hair to actively contribute to the ease of styling and/or holding of a hair set, and to maintain the restylability of the hair set. Hence, "hair setting compositions" include hair styling, hair fixative, and hair grooming products that conventionally are applied to the hair (wet or dry) in the form of gels, rinses, emulsions (oil-in-water, water-in-oil or multiphase), such as lotions and creams, pomades, sprays (pressurized or non-pressurized), spritzes, foams, such as mousses, shampoos, solids, such as sticks, semisolids and the like, or are applied from a hair setting aid having the hair setting composition impregnated therein or coated thereon, to leave the hair setting agent in contact on the hair for some period until removed, as by washing.

The term "hair setting composition" encompasses products comprising at least one surfactant-polymer blend of the present invention as a hair setting agent or as an adjuvant in combination with an auxiliary hair fixative agent, which is applied to the hair (wet or dry) before, during or after configuring the hair into the shape (curly or straight) desired, without limitation as to product form.

The term "conditioning agents," and grammatical variations thereof, as it relates to compositions for skin care and hair care includes cosmetically and pharmaceutically useful materials that are humectants, moisturizers, and emollients. It is recognized that some conditioning agents can serve more than one function in a composition, such as emulsifying agents, lubricants, and solvents.

The surfactant-polymer blends of the present invention are surprisingly useful in hair setting and hair styling compositions as the sole film-forming, rheology modifying, conditioning, and fixative agent. The surfactant-polymer blends of the present invention are also useful in combination with commercially available auxiliary hair fixative polymers, such as nonionic, cationic, and amphoteric hair setting polymers, cationic conditioning polymers, and combinations thereof. It is surprisingly found that unexpectedly increased viscosity and hair setting efficacy properties are produced by appropriate combinations of a polymer of the present invention with an auxiliary conventional hair fixative and/or hair conditioning polymer. Conventional polymeric hair fixative and hair styling polymers, well known in the art, include natural gums and resins and neutral or anionic polymers of synthetic origin. Listings of commercially available hair fixative and conditioning fixative polymers can be readily found in the INCI Dictionary, in supplier websites, and in the trade literature. See, for example, the Polymer Encyclopedia published in Cosmetics & Toiletries®, 117(12), December 2002 (Allured Publishing Corporation, Carol Stream, III.).

Suitable commercially available auxiliary fixatives include, but are not limited to, nonionic, cationic, anionic, and amphoteric polymers, as well as combinations thereof and include without limitation, polyvinylpyrrolidone (PVP), polyvinyl-pyrrolidone/vinylacetate copolymer (PVP/VA), and the like. Commercially available cationic fixative polymers include, without limitation thereto, polymers having the INCI name, polyquaternium, such as polyquaternium-4, a diallyldimonium chloride/hydroxy-ethylcellulose copolymer (such as CELQUAT^{®} H-100, National Starch); polyquaternium-11, a quaternized vinyl pyrrolidone/dimethylaminoethyl methacrylate copolymer (such as GAFQUAT^{®} 734, 755, 755N, ISP); polyquaternium-16, a quaternized vinyl pyrrolidone/vinylimidazolium chloride copolymer (such as LUVIQUAT^{®} FC-370, BASF); polyquaternium-28, a vinylpyrrolidone/methacrylamidopropyltrimethylammonium chloride copolymer (such as GAFQUAT^{®} HS-100, ISP); polyquaternium-46, a quaternized vinylcaprolactam/vinylpyrrolidone/methylvinyl-imidazolium methosulfate copolymer; polyquaternium-55, a quaternized vinyl-pyrrolidone/dimethylaminopropylmethylacrylamide/lauryldimethylpropylmethacrylamido-ammonium chloride copolymer (such as STYLEZE™ W, ISP), and the like; and amino-substituted polymers which are cationic under acidic pH conditions, such as vinylcaprolactam/PVP/dimethylaminoethylmethacrylate copolymer (such as GAFFIX^{®} VC-713, ISP); PVP/dimethylaminoethylmethacrylate copolymer (such as Copolymer 845, ISP), PVP/DMAPA acrylates copolymer (such as STYLEZE™ CC-10, ISP), the pyrrolidone carboxylic acid salt of chitosan, having the INCI name, Chitosan PCA (such as KYTAMER^{®} PC, Amerchol), and the like.

Additional auxiliary fixatives can be selected from one or more of the following polymers. Suitable commercially available nonionic polymers (i.e., neutral) used as hair styling or fixative polymers include, without limitation thereto, polyvinylpyrrolidone (PVP), polyvinylpyrrolidone/vinylacetate copolymer (PVP/VA), and the like. Commercially available cationic fixative polymers include, without limitation thereto, polymers having the INCI name, polyquaternium, such as polyquaternium-4, a diallyldimonium chloride/hydroxyethylcellulose copolymer (such as CELQUAT^{®} H-100, National Starch); polyquaternium-11, a quaternized vinyl pyrrolidone/dimethylaminoethyl methacrylate copolymer (such as GAFQUAT^{®} 734, 755, 755N, ISP); polyquaternium-16, a quaternized vinyl pyrrolidone/vinylimidazolium chloride copolymer (such as LUVIQUAT^{®} FC-370, BASF); polyquaternium-28, a vinylpyrrolidone/methacryl-amidopropyltrimethylammonium chloride copolymer (such as GAFQUAT^{®} HS-100, ISP); polyquaternium-46, a quaternized vinylcaprolactam/vinylpyrrolidone/methylvinyl-imidazolium methosulfate copolymer; polyquaternium-55, a quaternized vinyl-pyrrolidone/dimethylaminopropylmethylacrylamide/lauryldimethylpropylmethacryl amido-ammonium chloride copolymer (such as STYLEZE™ W, ISP), and the like; and amino-substituted polymers which are cationic under acidic pH conditions, such as vinylcaprolactam/PVP/dimethylaminoethylmethacrylate copolymer (such as GAFFIX^{®} VC-713, ISP); PVP/dimethylaminoethylmethacrylate copolymer (such as Copolymer 845, ISP), PVP/DMAPA acrylates copolymer (such as STYLEZE™ CC-10, ISP), the pyrrolidone carboxylic acid salt of chitosan, having the INCI name, Chitosan PCA (such as KYTAMER^{®} PC, Amerchol), and the like. Suitable amphoteric fixative polymers include, without limitation thereto, octylacryamide/acrylates/butylaminoethylmethacrylate copolymer (such as the AMPHOMER^{®} polymers, National Starch), acrylates/lauryl acrylate/stearyl acrylate/ethylamine oxide methacrylate copolymers (such as the DIAFORMER^{®} polymers, Clariant Corp.), and the like. acrylamide/acrylates/butyl-aminoethylmethacrylate copolymer (such as the AMPHOMER^{®} polymers, National Starch), acrylates/lauryl acrylate/stearyl acrylate/ethylamine oxide methacrylate copolymers (such as the DIAFORMER^{®} polymers, Clariant Corp.), and the like.

Additional fixative polymers that can be utilized with the inventive polymers of the invention or in combination with one or more of the foregoing auxiliary fixatives, include without limitation, one or more of the following fixative polymers: polyoxythylenated vinyl acetate/crotonic acid copolymers, vinyl acetate crotonic acid copolymers, vinyl methacrylate copolymers, monoalkyl esters of poly(methyl vinyl ether (PVM)/maleic acid (MA)), such as, for example, ethyl, butyl and isopropyl esters of PVM/MA copolymer acrylic acid/ethyl acrylate/N-tert-butyl-acrylamide terpolymers, and poly (methacrylic acid/acrylamidomethyl propane sulfonic acid), acrylates copolymer, acrylates/octylacrylamide copolymer, vinyl acetate (VA)/crotonates/vinyl neodeanoate copolymer, poly(N-vinyl acetamide), poly(N-vinyl formamide), corn starch modified, sodium polystyrene sulfonate, polyquaterniums such as, for example, Polyquaternium-24, Polyquaternium-29, Polyquaternium-32, Polyquaternium-34, Polyquaternium-37, Polyquaternium-39, Polyquaternium-44, Polyquaternium-47, Polyquaternium-69, Polyquaternium-87, polyether-1, polyurethanes, VA/acrylates/lauryl methacrylate copolymer, adipic acid/dimethylaminohydroxypropyl diethylene AMP/acrylates copolymer, methacrylol ethyl betaine/acrylates copolymer, VP/methacrylamide/vinyl imidazole copolymer, VP/vinylcaprolactam/DMAPA acrylates copolymer, VP/dimethylaminoethylmethacrylate copolymer, VP/DMAPA acrylates copolymer, vinyl caprolactam/VP/dimethylaminoethyl methacrylate copolymer, VA/butyl maleate/isobornyl acrylate copolymer, VA/crotonates copolymer, acrylate/acrylamide copolymer, VA/crotonates/vinyl propionate copolymer, VP/vinyl acetate/vinyl propionate terpolymers, VA/crotonates, VP/vinyl acetate copolymer, VP/acrylates copolymer, VA/crotonic acid/vinyl proprionate, acrylates/acrylamide, acrylates/octylacrylamide, acrylates/hydroxyacrylates copolymer, acrylates/hydroxyesteracrylates copolymer, acrylates/stereth-20 methacrylate copolymer, tert-butyl acrylate/acrylic acid copolymer, diglycol/cyclohexanedimethanol/isophthalates/sulfoisophthalates copolymer, VA/butyl maleate and isobornyl acrylate copolymer, vinylcaprolactam/VP/dimethylaminoethyl methacrylate, VA/alkylmaleate half ester/N-substituted acrylamide terpolymers, vinyl caprolactam/VP/ methacryloamidopropyl trimethylammonium chloride terpolymer, methacrylates/acrylates copolymer/amine salt, polyvinylcaprolactam, polyurethanes, hydroxypropyl guar, poly (methacrylic acid/acrylamidomethyl propane sulfonic acid (AMPSA), ethylenecarboxamide (EC)/AMPSA/methacrylic acid (MAA), poylurethane/acrylate copolymers and hydroxypropyl trimmonium chloride guar, acrylates copolymer, acrylates crosspolymer, AMP-acrylates/allyl methacrylate copolymer, polyacrylate-14, polyacrylate-2 crosspolymer, octylacryl-amide/acrylates/butylaminoethyl methacrylate copolymer, acrylates/octylacrylamide copolymer, VA/crotonates/vinyl neodeanoate copolymer, poly(N-vinyl acetamide), poly(N-vinyl formamide), polyurethane, methacryloyl ethyl betaines/methacrylates copolymer, corn starch modified, sodium polystyrene sulfonate, polyurethane/acrylates copolymer, chitosan glycolate, cationic polygalactomannans, such as, for example, quaternized derivatives of guar and cassia, such as, for example, guar hydroxypropyl trimmonium chloride, hydroxypropyl guar hydroxypropyl trimmonium chloride and cassia hydroxypropyl trimmonium chloride. Many of the foregoing polymers are referred to by their INCI nomenclature set forth in the International Cosmetic Ingredient Dictionary published by the Cosmetic, Toiletry, and Fragrance Association, Washington D.C. Other suitable auxiliary fixative polymers are disclosed in United States Patent No. 7,205,271.

The surfactant-polymer blends of the invention provide desirable rheological properties to aqueous personal care, health care, home care, and "I&I" products. The surfactant-polymer blends of the present invention beneficially can thicken aqueous formulations to provide aesthetically smooth-textured products that flow smoothly and spread easily. The form of the surfactant-polymer containing product can range from a non-pourable, stiff to soft gel, a semisolid paste to a substantially solid stick or bar, and aerosolized foam to squeezable gel, as well as a non-runny, yet flowable, product, suitable for pumpable spray or roll-on products and liquid lotions.

In one embodiment, the surfactant-polymer blend is added to a desired personal care, health care, home care, and "I&I" formulation and the pH is adjusted upward with an alkaline material such as an organic base to optimize swelling of the polymer in the blend to the desired viscosity, and then adjusting the final composition to the desired pH. If the pH of a completed composition or formulation containing the surfactant-polymer blend is more alkaline than required for the intended use of the formulation, the pH can then be further adjusted with any, physiologically tolerable, inorganic or organic acid.

Antistatic agents include, but are not limited to, quaternary ammonium compounds, protein derivatives, synthetic quaternary ammonium polymers, amines, protonated amine oxides, betaines, and the like, which may act as antistatic agents in specific formulations and under controlled pH conditions in addition to any surfactant properties imparted by such materials. In addition to antistatic agents previously discussed, non-limiting examples of quaternary ammonium compounds useful as antistatic agents are acetamidopropyl trimonium chloride, behenamidopropyl dimethylamine, behenamidopropyl ethyldimonium ethosulfate, behentrimonium chloride, cetethyl morpholinium ethosulfate, cetrimonium chloride, cocoamidopropyl ethyldimonium ethosulfate, dicetyldimonium chloride, dimethicone hydroxypropyl trimonium chloride, hydroxyethyl behenamidopropyl dimonium chloride, quaternium-26, quaternium-27, quaternium-53, quaternium-63, quaternium-70, quaternium-72, quaternium-76 hydrolyzed collagen, PPG-9 diethylmonium chloride, PPG-25 diethylmonium chloride, PPG-40 diethylmonium chloride, stearalkonium chloride, stearamidopropyl ethyl dimonium ethosulfate, steardimonium hydroxypropyl hydrolyzed wheat protein, steardimonium hydroxypropyl hydrolyzed collagen, wheat germamidopropalkonium chloride, wheat germamidopropyl ethyldimonium ethosulfate, and the like.

Synthetic quaternary ammonium polymers, include film-forming polymers and conditioning polymers. Non-limiting examples of synthetic quaternary ammonium polymers include polymers and copolymers of dimethyl diallyl ammonium chloride, such as polyquaternium-4, polyquaternium-6, polyquaternium-7, polyquaternium-22, polyquaternium-10, polyquaternium-11 polyquaternium-15, polyquaternium-16, polyquaternium-24, polyquaternium-28, polyquaternium-32, polyquaternium-33, polyquaternium-35, polyquaternium-37, polyquaternium-39, polyquaternium-43, polyquaternium-44, PEG-2-cocomonium chloride, and cassia hydroxypropyltrimonium chloride, quaternium-52, polyquaternium-46, polyquaternium-55, polyquaternium-44, polyquaternium-60, polyquaternium-66, polyquaternium-67, polyquaternium-68, polyquaternium-69, polyquaternium-72, polyquaternium-77, polyquaternium-85, polyquaternium-86, polyquaternium-87, and the like.

Suitable commercial conditioning polymers include polymeric quaternary ammonium salts such as, without being limited thereto, polyquaternium-7, a polymeric quaternary ammonium salt of acrylamide and dimethyl diallylammonium chloride monomers (such as MACKERNIUM™-007, McIntyre Group, Ltd.); polyquaternium-10, a polymeric quaternary ammonium salt of hydroxyethylcellulose reacted with a trimethylammonium substituted epoxide (such as the UCARE^{®} Polymers JR, LK, LR, SR series, Amerchol and CELQUAT^{®} SC series, National Starch); polyquaternium-39, a polymeric quaternary ammonium salt of acrylic acid, diallyl dimethylammonium chloride and acrylamide (such as the MERQUAT^{®} and MERQUAT^{®} Plus polymers, Ondeo Nalco); quaternized derivatives of natural gums, e.g., guar hydroxypropyltrimonium chloride (such as the JAGUAR^{®} and JAGUAR^{®} Excel polymers, Rhodia, Inc.), and the like.

A number of non-polymeric quaternary ammonium compounds are used for fabric conditioning and fabric care, generally referred to as fabric softening agents, and are typically employed in amounts of up to about 20 weight percent of the total weight of the formulation, but are not limited thereto. Fabric softening agents useful in combination with the surfactant-polymer blend compositions of the present invention generally include long-chain alkylated quaternary ammonium compounds such as dialkyldimethyl quaternary ammonium compounds, imidazoline quaternary compounds, amidoamine quaternary compounds, dialkyl ester quat derivatives of dihydroxypropyl ammonium compounds; dialkyl ester quat derivatives of methyltriethanol ammonium compounds, ester amide amine compounds, and diester quat derivatives of dimethyldiethanol ammonium chloride, as described in the review article by Whalley, Fabric Conditioning Agents, HAPPI, pp. 55 to 58 (February 1995), incorporated herein by reference.

In addition to the previously discussed antistatic agents, non-limiting examples of dialkyldimethyl quaternary ammonium compounds, include N,N-dioleyl-N,N-dimethylammonium chloride, N,N-ditallowyl-N,N-dimethylammonium ethosulfate, N,N-di(hydrogenated-tallowyl)-N,N-dimethylammonium chloride, and the like. Non-limiting examples of imidazoline quaternary compounds include 1-N-methyl-3-N-tallowamidoethylimidazolium chloride, 3-methyl-1-tallowylamidoethyl-2-tallowyl-imidazolinium methylsulfate, available from Witco Chemical Company under the tradename VARISOFT^{®} 475, and the like. Non-limiting examples of amidoamine quaternary compounds include N-alkyl-N-methyl-N,N-bis(2-tallowamidoethyl)ammonium salts where the alkyl group can be methyl, ethyl, hydroxyethyl, and the like. Non-limiting examples of dialkyl ester quat derivatives of dihydroxypropyl ammonium compounds include 1,2-ditallowoyloxy-3-N,N,N-trimethylammoniopropane chloride, 1,2-dicanoloyloxy-3-N,N,N-trimethylammoniopropane chloride, and the like.

In addition, other types of long chain (e.g., natural oil and fatty acid-derived) alkylated quaternary ammonium compounds are suitable fabric softening agents, including, but not limited, to N,N-di(alkyloxyethyl)-N,N-dimethylammonium salts such as N,N-di(tallowyloxyethyl)-N,N-dimethylammonium chloride, N,N-di(canolyloxyethyl)-N,N-dimethylammonium chloride, and the like; N,N-di(alkyloxyethyl)-N-methyl-N-(2-hydroxyethyl)ammonium salts such as N,N-di(tallowyloxyethyl)-N-methyl-N-(2-hydroxyethyl)ammonium chloride, N,N-di(canolyloxyethyl)-N-methyl-N-(2-hydroxyethyl)ammonium chloride, and the like; N,N-di(2-alkyloxy-2-oxoethyl)-N,N-dimethylammonium salts, such as N,N-di(2-tallowyloxy-2-oxoethyl)-N,N-dimethylammonium chloride, N,N-di(2-canolyloxy-2-oxoethyl)-N,N-dimethylammonium chloride, and the like; N,N-di(2-alkyloxyethylcarbonyloxyethyl)-N,N-dimethylammonium salts, such as N,N-di(2-tallowyloxyethylcarbonyloxyethyl)-N,N-dimethylammonium chloride, N,N-di(2-canolyloxyethylcarbonyloxyethyl)-N,N-dimethylammonium chloride, and the like; N-(2-alkanoyloxy-2-ethyl)-N-(2-alkyloxy-2-oxoethyl)-N,N-dimethyl ammonium salts, such as N-(2-tallowoyloxy-2-ethyl)-N-(2-tallowyloxy-2-oxoethyl)-N,N-dimethyl ammonium chloride, N-(2-canoloyloxy-2-ethyl)-N-(2-canolyloxy-2-oxoethyl)-N,N-dimethyl ammonium chloride, and the like; N,N,N-tri(alkyloxyethyl)-N-methyl ammonium salts, such as N,N,N-tri(tallowyloxyethyl)-N-methylammonium chloride, N,N,N-tri(canolyloxyethyl)-N-methylammonium chloride, and the like; N-(2-alkyloxy-2-oxoethyl)-N-alkyl-N,N-dimethyl ammonium salts, such as N-(2-tallowyloxy-2-oxoethyl)-N-tallowyl-N,N-dimethyl ammonium chloride, N-(2-canolyloxy-2-oxoethyl)-N-canolyl-N,N-dimethyl ammonium chloride, and the like.

In one embodiment, the long-chain alkyl groups are derived from tallow, canola oil, or from palm oil, however, other alkyl groups derived from soybean oil and coconut oil, for example, are also suitable, as are lauryl, oleyl, ricinoleyl, stearyl, palmityl, and like fatty alkyl groups. The quaternary ammonium salt compounds can have any anionic group as a counter-ion, for example, chloride, bromide, methosulfate (i.e., methylsulfate), acetate, formate, sulfate, nitrate, and the like.

Examples of suitable quaternary ammonium fabric softening compounds include N-methyl-N,N-bis(tallowamidoethyl)-N-(2-hydroxyethyl)ammonium methylsulfate and N-methyl-N,N-bis(hydrogenated-tallowamidoethyl)-N-(2-hydroxyethyl) ammonium methylsulfate, each of which materials are available from Witco Chemical Company under the trade names VARISOFT^{®} 222 and VARISOFT^{®} 110, respectively; dialkyl esterquat derivatives of methyltriethanol ammonium salts such as the DEHYQUART^{®} AU series of bis(acyloxyethyl) hydroxyethylmethylammonium methosulfate esterquats available from Cognis, such as DEHYQUART^{®} AU35, AU46, AU56, and the like; and N,N-di(tallowoyloxyethyl)-N,N-dimethylammonium chloride, where the tallow chains are at least partially unsaturated. Other suitable fabric softening agents include the well-known dialkyldimethyl ammonium salts such as N,N-ditallowyl-N,N-dimethyl ammonium methylsulfate, N,N-di(hydrogenated-tallowyl)-N,N-dimethyl ammonium chloride, N,N-distearyl-N,N-dimethyl ammonium chloride, N,N-dibehenyl-N,N-dimethylammonium chloride, N,N-di(hydrogenated tallow)-N,N-dimethyl ammonium chloride (trade name ADOGEN^{®} 442), N,N-ditallowyl-N,N-dimethyl ammonium chloride (trade name ADOGEN^{®} 470, PRAEPAGEN^{®} 3445), N,N-distearyl-N,N-dimethyl ammonium chloride (trade name AROSURF^{®} TA-100), all available from Witco Chemical Company; N,N-dibehenyl-N,N-dimethyl ammonium chloride, sold under the trade name KEMAMINE^{®} Q-2802C by Humko Chemical Division of Witco Chemical Corporation; and N,N-dimethyl-N-stearyl-N-benzylammonium chloride sold under the trade names VARISOFT^{®} SDC by Witco Chemical Company and AMMONYX^{®} 490 by Onyx Chemical Company.

Any of the foregoing fabric softening agents, and mixtures thereof, can be utilized in combination with the surfactant-polymer blend compositions of the present invention, particularly in laundry and fabric care products. For ester-containing fabric softening agents, the pH of the compositions can influence the stability of the fabric softening agents, especially in prolonged storage conditions. The pH, as defined in the present context, is measured in the neat compositions at 20°C. In one embodiment, the pH of the composition is less than 6. For optimum hydrolytic stability of these compositions, the pH is in the range of from 2 to 5, or from 2.5 to 3.5.

In addition to protein derivatives previously described, non-limiting examples of protein derivatives include cocodimonium hydroxypropyl hydrolyzed casein, cocodimonium hydroxypropyl hydrolyzed collagen, cocodimonium hydroxypropyl hydrolyzed hair keratin, cocodimonium hydroxypropyl hydrolyzed rice protein, cocodimonium hydroxypropyl hydrolyzed silk, cocodimonium hydroxypropyl hydrolyzed soy protein, cocodimonium hydroxypropyl hydrolyzed wheat protein, cocodimonium hydroxypropyl hydrolyzed silk amino acids, hydroxypropyl trimonium hydrolyzed collagen, hydroxypropyl trimonium hydrolyzed keratin, hydroxypropyl trimonium hydrolyzed silk, hydroxypropyl trimonium hydrolyzed rice bran, hydroxypropyl trimonium hydrolyzed soy protein, hydroxypropyl trimonium hydrolyzed vegetable protein, hydroxypropyl trimonium hydrolyzed wheat protein, soyethyldimonium ethosutfate, soyethyl morpholinium ethosulfate, and the like.

The surfactant-polymer blends of the invention are useful as a thickener for antistatic, biocidal, antimicrobial, and other preservative compositions, in a variety of personal care, health care, I&I, and medical applications. For example, the polymer can be employed as a thickener in over-the-counter (OTC) health care and pharmaceutical products where cationic biocides are typically employed, such as in oral care compositions for plaque and tartar control, and liquid vehicles containing therapeutic agents, such as syrups, gels, and the like.

Products containing a surfactant-polymer blend of the invention can be packaged and dispensed from containers, such as jars, bottles, tubes, spray bottles, wipes, cans, roll-on containers, stick containers, and the like, without limitation. There is no limitation as to the form of product in which the surfactant-polymer can be incorporated, so long as the purpose for which the product is used is achieved. For example, personal care and health care products containing a surfactant-polymer can be applied to the skin, hair, scalp and nails in the form of, without being limited thereto, gels, mousses, sprays (liquid or foam), emulsions (creams, lotions, pastes), liquids (rinses, shampoos), bars, ointments, suppositories, impregnated wipes, patches, and the like.

### Additives and Adjuvants:

Product formulations comprising the surfactant-polymer blends of this invention can contain the various additives and adjuvants previously described and/or conventionally or popularly included in personal care, health care, home care, "I&I" care products, and in industrial processes, including, without being limited thereto, acidifying or alkalizing pH adjusting agents and buffering agents; auxiliary fixatives and film formers (e.g., nonionic, anionic, cationic, or amphoteric polymers of synthetic or natural origin); auxiliary rheology modifiers (e.g., viscosity-increasing polymeric, gum, or resin thickeners or gellants); additives (e.g., auxiliary, auxiliary emulsifiers, auxiliary emulsion stabilizers, waxes, auxiliary dispersants, and the like), viscosity control agents (e.g., electrolytes), auxiliary conditioning agents (e.g., synthetic oils, natural oils, animal oils, natural and synthetic waxes, silicones, monomeric and polymeric quaternized ammonium compounds (previously described) and derivatives thereof), sheen enhancers; moisturizers; emollients; humectants; lubricants; sunscreen agents; UV absorbing agents; exidizing agents; reducing agents; surfactants (e.g., anionic, cationic, nonionic, amphoteric, zwitterionic, and silicone derivatives thereof, and mixtures thereof); polymer film modifying agents (e.g., plasticizers, tackifiers, de-tackifiers, wetting agents, and the like); chelating agents; opacifiers; pearlescing agents; proteinaceous materials and derivatives thereof; vitamins and derivatives thereof; botanicals; antifungal agents; antidandruff agents; anti-inflammatory agents; analgesics; preservatives; fragrances; fragrance solubilizers; colorants (e.g., pigments and dyes); propellants (e.g., fluorinated hydrocarbons, liquid volatile hydrocarbons, compressed gases, and the like); and mixtures thereof.

Additives and adjuvant ingredients, products, or materials, which may be employed with the inventive surfactant-polymer blend compositions discussed herein are in some cases referred to by the international nomenclature commonly referred to as INCI name given them in the International Cosmetic Ingredient Dictionary, published by the Cosmetic, Toiletry, and Fragrance Association, Washington D.C. (see www.ctfa-online.org - hereafter INCI Dictionary), such as can be found in any edition thereof, for example, Volumes 1 and 2, Sixth Edition, (1995) or Volumes 1-3, Seventh and Eighth Editions, (1997, 2000), or by their commonly used chemical names. Numerous commercial suppliers of materials listed by INCI name, trade name or both can be found in the INCI Dictionary and in numerous commercial trade publications, including but not limited to the 2001 McCutcheon 's Directories, Volume 1: Emulsifiers & Detergents and Volume 2: Functional Materials, published by McCutcheon's Division, The Manufacturing Confectioner Publishing Co., Glen Rock, N.J. (2001); and 2001 Cosmetic Bench Reference, edition of Cosmetics & Toiletries® 115 (13), published by Allured Publishing Corporation, Carol Stream, III. (2001). Such components and the formulation of compositions are also described in detail in well known references, such as Cosmetics Science and Technology, First Edition (Sagarin (ed)), published 1957, and Second Edition (Balsam, et al. (eds)), published 1972 to 1974; and The Chemistry and Manufacture of Cosmetics, Second Edition (deNavarre (ed)), published 1975, and Third Edition (Schlossman (ed)), published 2000, both available from Allured Publishing Corporation; Rieger (ed), Harry's Cosmeticology, 8th Edition, Chemical Publishing, Co., Inc., New York, N.Y. (2000); and various formularies available to those skilled in the pharmaceutical arts, such as Remington's Pharmaceutical Sciences, Fourteenth Edition, Mack Publishing Company, Easton, Pa. (1970).

It is known that formulated compositions for personal care and topical, dermatological, health care, which are applied to the skin and mucous membranes for cleansing or soothing, are compounded with many of the same or similar physiologically tolerable ingredients and formulated in the same or similar product forms, differing primarily in the purity grade of ingredient selected, by the presence of medicaments or pharmaceutically accepted compounds, and by the controlled conditions under which products may be manufactured. Likewise, many of the ingredients employed in products for home care and I&I are the same or similar to the foregoing, differing primarily in the amounts and material grade employed. It is also known that the selection and permitted amount of ingredients also may be subject to governmental regulations, on a national, regional, local, and international level. Thus, discussion herein of various useful ingredients for personal care and health care products may apply to home care and I&I products and industrial applications.

### Solvents:

The polymers of the present invention prepared as aqueous emulsions are particularly useful for water-based formulations, and formulations containing water-miscible auxiliary solvents, but are not limited thereto. Useful solvents commonly employed are typically liquids, such as water (deionized, distilled or purified), alcohols, polyols, and the like, and mixtures thereof. Non-aqueous or hydrophobic auxiliary solvents are commonly employed in substantially water-free products, such as nail lacquers, aerosol propellant sprays, or for specific functions, such as removal of oily soils, sebum, make-up, or for dissolving dyes, fragrances, and the like, or are incorporated in the oily phase of an emulsion. Non-limiting examples of auxiliary solvents, other than water, include linear and branched alcohols, such as ethanol, propanol, isopropanol, hexanol, and the like; aromatic alcohols, such as benzyl alcohol, cyclohexanol, and the like; saturated C₁₂ to C₃₀ fatty alcohol, such as lauryl alcohol, myristyl alcohol, cetyl alcohol, stearyl alcohol, behenyl alcohol, and the like. Non-limiting examples of polyols include polyhydroxy alcohols, such as glycerin, propylene glycol, butylene glycol, hexylene glycol, C₂ to C₄ alkoxylated alcohols and C₂ to C₄ alkoxylated polyols, such as ethoxylated, propoxylated, and butoxylated ethers of alcohols, diols, and polyols having 2 to 30 carbon atoms and 1 to about 40 alkoxy units, polypropylene glycol, polybutylene glycol, and the like. Non-limiting examples of non-aqueous auxiliary solvents include silicones, and silicone derivatives, such as cyclomethicone, and the like, ketones such as acetone and methylethyl ketone; natural and synthetic oils and waxes, such as vegetable oils, plant oils, animal oils, essential oils, mineral oils, C₇ to C₄₀ isoparaffins, alkyl carboxylic esters, such as ethyl acetate, amyl acetate, ethyl lactate, and the like, jojoba oil, shark liver oil, and the like. Some of the foregoing non-aqueous auxiliary solvents may also be conditioners and emulsifiers.

### Propellants:

Suitable propellants that can be utilized in compositions comprising the surfactant-polymer blend compositions of the invention include but are not limited to Where applicable, any known aerosol propellant can be utilized to deliver the personal care, home care, health care and institutional care compositions containing the esters of the present invention in combination with one or more of the foregoing active ingredients and/or with the one or more additives and/or adjuvants, conventionally or popularly included in personal care, health care, home care, and institutional care products discussed above. Exemplary propellants include, but are not limited to, lower boiling hydrocarbons such as C₃ to C₆ straight and branched chain hydrocarbons. Exemplary hydrocarbon propellants include propane, butane, isobutene, and mixtures thereof. Other suitable propellants include ethers, such as, dimethyl ether, hydrofluorocarbons, such as, 1,1-difluoroethane, and compressend gasses, such as air and carbon dioxide. These compositions can contain from 0.5 weight percent to 60 weight percent of the propellant in one embodiment and from 0.5 weight percent to 35 weight percent in another embodiment, based on the total weight of the composition.

### pH Adjusting Agents:

Suitable acidic pH adjusting agents are selected from organic acids, including amino acids, and inorganic mineral acids. Examples of acidic pH adjusting agents include, but are not limited to, acetic acid, salicylic acid, citric acid, fumaric acid, glutamic acid, glycolic acid, hydrochloric acid, lactic acid, nitric acid, phosphoric acid, sodium bisulfate, sulfuric acid, tartaric acid, and the like, and mixtures thereof. Suitable alkaline or basic pH adjusting agents can be added to swell the polymer contained in the surfactant-polymer blend of the invention. Examples of alkaline pH adjusting agents include, but are not limited to, alkali metal hydroxides, such as sodium hydroxide, and potassium hydroxide; ammonium hydroxide; organic bases, such as triethanolamine, diisopropylamine, dodecylamine, diisopropanolamine, aminomethyl propanol, cocamine, oleamine, morpholine, triamylamine, triethylamine, tromethamine (2-amino-2-hydroxymethyl)-1,3-propanediol), and tetrakis(hydroxypropyl)ethylene-diamine; and alkali metal salts of inorganic acids, such as sodium borate (borax), sodium phosphate, sodium pyrophosphate, and the like, and mixtures thereof. The acidic and alkaline pH adjusting agent can be utilized in any amount necessary to obtain a desired pH value in the final composition.

### Silicones:

Silicones are commonly used in shampoo products, such as the so-called "two-in-one" combination cleansing/conditioning shampoos, and in skin care products as well. Silicones provide conditioning properties as well as psychosensory and aesthetic properties to a formulation in which they are included, making the hair and skin feel softer and smoother to the touch. Silicones can also functions as emulsifiers and as emollients in a personal care formulation. The most common class of silicone polymers are the linear polydimethyl siloxanes having the general formula (CH₃)₃-Si(CH₃)₂-O-(Si(CH₃)₂-O)_{w}-Si(CH₃)₃ where w is an integer greater than 2. Silicones can also be branched materials wherein one or more alkyl groups in a polymer are replaced with oxygen to create a branch point. The silicon atoms may carry a wide variety of substituents which can be the same or different. The chemically least reactive substituents are the methyl or phenyl groups. Functional endblocking groups may carry nitrogen or hydroxyl moieties, as in the case of dimethiconol. Background material on silicones including sections discussing silicone fluids, gums, and resins, as well as manufacture of silicones, are found in Encyclopedia of Polymer Science and Engineering, Vol. 15, 2d ed., pp. 204 to 308, John Wiley & Sons, Inc. (1989).

Silicone fluids are typically water-insoluble oils having a viscosity in the range of a few mPa·s to several hundred thousand mPa·s. They are in the form of fluids (volatile and non-volatile), gums, gums in fluids, resins or in the form of non-ionic small and large particle size emulsions (MEM 1664, MEM 1310, 5-7137, 2-1352, MEM 1784, MEM 1310, MEM 1491, 5-7137, and MEM 2220 from Dow Corning), and anionic emulsions (MEM 1784 from Dow Corning). The refractive index of the polysiloxane fluid will generally be less than 1.70, typically less than 1.60. In this context, polysiloxane "fluid" includes oils as well as gums.

Another class of silicones for use in hair care products are the so-called rigid silicones (also known as silicone gums), as described, for example in United States Patent No. 4,902,499 which generally have a viscosity (at 20°C) of greater than 600,000 mPa·s and have a weight average molecular weight of at least 500,000 Daltons as determined by intrinsic viscosity measurement. Gums are also described in United States Patent No. 4,152,416; Noll and Walter, Chemistry and Technology of Silicones, New York: Academic Press (1968); and in General Electric Silicone Rubber Product Data Sheets SE 30, SE 33, SE 54 and SE 76. Specific non-limiting examples of gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

Volatile silicones are particularly useful in combination with the polymers of the present invention and are often used as lubricants in hair care products, such as shampoos. Volatile silicones include cyclic and linear polydimethylsiloxanes, and the like. Cyclic volatile silicones typically contain 3 to 7 silicon atoms, alternating with oxygen atoms, in a cyclic ring structure. Each silicon atom is also substituted with two alkyl groups, typically methyl groups. Linear volatile silicones are silicone fluids, as described above, having viscosities of not more than 25 mPa·s. A description of volatile silicones is found in Todd and Byers, Volatile Silicone Fluids for Cosmetics, Cosmetics and Toiletries, Vol. 91(1), pp. 27 to 32 (1976), and in Kasprzak, Volatile Silicones, Soap/Cosmetics/Chemical Specialties, pp. 40 to 43 (December 1986).

Another class of silicones includes aminosilicone category which contains any amine functionalized silicone; i.e., a silicone containing at least one primary amine, secondary amine, tertiary amine, or a quaternary ammonium group. Aminosilicones can be graft or terminal. In one embodiment, the aminosilicone has a viscosity of from 1,000 •10⁻⁶ m²/s (cs) to 1,000,000 •10⁻⁶ m²/s (cs), or from 2,000 •10⁻⁶ m²/s (cs) to 600,000 •10⁻⁶ m²/s (cs), or even from 4,000 •10⁻⁶ m²/s (cs) to 400,000•10⁻⁶ m²/s (cs). Examples of aminosilicone fluids with low and high amine content (8500, 2-8566, AP-8087 from Dow Coming); amino glycol copolymer; amino phenyl resin; low and high viscosity Cationic emulsions (949, 2-8194 from Dow Corning), nonionic microemulsions; silicone quat microemulsions (5-7113 from Dow Corning), and mixtures of any two or more thereof.

Other silicone oils include the dimethicone copolyols, which are linear or branched copolymers of dimethylsiloxane (dimethicone) and alkylene oxides. The dimethicone polyols can be random or block copolymers. A generally useful class of dimethicone polyols are block copolymers having blocks of polydimethylsiloxane and blocks of polyalkylene oxide, such as blocks of polyethylene oxide, polypropylene oxide, or both. Dimethicone copolyols are disclosed in United States Patent Nos. 5,136,063 and 5,180,843. In addition, dimethicone copolyols are commercially available under the Silsoft^{®} and Silwet^{®} brand names from the General Electric Company (GE-OSi). Specific product designations include but are not limited to Silsoft 305, 430, 475, 810, 895, Silwet L 7604 (GE-OSi); Dow Corning^{®} 5103 and 5329 from Dow Coming Corporation; and Abil^{®} dimethicone copolyols, such as, for example WE 09, WS 08, EM 90 and EM 97 from Evonik Goldschmidt Corporation; and Silsense™ dimethicone copolyols, such as Silsense Copolyol-1 and Silsense Copolyol-7, available from Lubrizol Advanced Materials, Inc.

Silicone materials, including volatile silicones, silicone gums, and silicone copolymers, mixtures of dimethicones and dimethiconols; phenyl-modified silicones, alkyl/alkoxy-modified silicones, polyether functional silicones, polyglycerin-modified silicones, polyether/alkyl-modified silicones, polyglycerin/alkyl-modified silicones, silicone cross-polymers, silicone resin, silicone resin gels, silicone polyglucosides, are available from a variety of commercial sources such as Dow Coming, Shin-Etsu, Wacker, General Electric Company, Momentive Performance Materials, and Lubrizol.

The silicones used in the present invention have a particle size of from 0.1 µm to 300 µm, or from 5 µm to 80 µm , or from 20 µm to 60 µm, or even from 30 µm to 50 µm.

### Conditioners:

In addition to the silicone and quaternary (monomeric and polymeric) conditioners previously disclosed, exemplary conditioners include synthetic oil conditioners selected from polyolefins, e.g., poly-α-olefins such as polybutenes, polyisobutenes and polydecenes. The polyolefins can be hydrogenated. Fluorinated or perfluronated oils are also contemplated within the scope of the present invention. Fluorinated oils include perfluoropolyethers described in European Patent 0 486 135 and the fluorohydrocarbon compounds described in WO 93/11103. The fluoridated oils may also be fluorocarbons such as fluoramines, e.g., perfluorotributylamine, fluoridated hydrocarbons, such as perfluorodecahydronaphthalene, fluoroesters, and fluoroethers.

Suitable natural oil conditioners include but are not limited to peanut, sesame, avocado, coconut, cocoa butter, almond, safflower, com, cotton seed, sesame seed, walnut oil, castor, olive, jojoba, palm, palm kernel, soybean, wheat germ, linseed, sunflower seed; eucalyptus, lavender, vetiver, litsea, cubeba, lemon, sandalwood, rosemary, chamomile, savory, nutmeg, cinnamon, hyssop, caraway, orange, geranium, cade, and bergamot oils, fish oils, glycerol tricaprocaprylate; and mixtures thereof.

Suitable natural and synthetic wax conditioning agents include but are not limited to carnauba wax, candelila wax, alfa wax, paraffin wax, ozokerite wax, olive wax, rice wax, hydrogenated jojoba wax, bees wax, modified bees wax, e.g., cerabellina wax, marine waxes, polyolefin waxes, e.g., polyethylene wax; and mixtures thereof.

In one aspect, the conditioning agent(s) can be present in an amount of 0.001 weight percent to 20 weight percent in one embodiment, from 0.01 weight percent to 10 weight percent in another embodiment, and from 0.1 weight percent to 3 weight percent in still yet another embodiment, based on the total weight of the composition.

### Emulsifiers:

Exemplary emulsifiers include but are not limited to C₁₂ to C₁₈ fatty alcohols; alkoxylated C₁₂ to C₁₈ fatty alcohols; C₁₂ to C₁₈ fatty acids; and alkoxylated C₁₂ to C₁₈ fatty acids, the alkoxylates each having 10 to 30 units of ethylene oxide, propylene oxide, and combinations of ethylene oxide/propylene oxide; C₈ to C₂₂ alkyl mono and oligoglycosides; ethoxylated sterols; partial esters of polyglycerols; esters and partial esters of polyols having 2 to 6 carbon atoms and saturated and unsaturated fatty acids having 12 to 30 carbon atoms; partial esters of polyglycerols; and organosiloxanes; and combinations thereof.

The fatty alcohols, acids and alkoxylated fatty alcohols and fatty acids are as described in the emollient description above. In one aspect of the invention the fatty alcohols and fatty acids each are ethoxylated with 10 to 30 units of ethylene oxide.

The C₈ to C₂₂ alkyl mono- and oligoglycoside emulsifiers are prepared by reacting glucose or an oligosaccharide with primary fatty alcohols having 8 to 22 carbon atoms. Products which are obtainable under the trademark Plantacare^{®} comprise a glucosidically bonded C₈ to C₁₆ alkyl group on an oligoglucoside residue whose average degree of oligomerization is 1 to 2. Exemplary alkyl glucosides and oligoglycosides are selected from octyl glucoside, decyl glucoside, lauryl glucoside, palmityl glucoside, isostearyl glucoside, stearyl glucoside, arachidyl glucoside and behenyl glucoside, and mixtures thereof.

Exemplary ethoxylated sterols include ethoxylated vegetable oil sterols such as, for example, soya sterols. The degree of ethoxylation is greater than 5 in one aspect, and at least 10 in another aspect. Suitable ethoxylated sterols are PEG-10 Soy Sterol, PEG-16 Soy Sterol and PEG-25 Soy Sterol.

The partial esters of polyglycerols have 2 to 10 glycerol units and are esterified with 1 to 4 saturated or unsaturated, linear or branched, optionally hvdroxylated C₈ to C₃₀ fatty acid residues. Representative partial esters of polyglycerols include diglycerol monocaprylate, diglycerol monocaprate, diglycerol monolaurate, triglycerol monocaprylate, triglycerol monocaprate, triglycerol monolaurate, tetraglycerol monocaprylate, tetraglycerol monocaprate, tetraglycerol monolaurate, pentaglycerol monocaprylate, pentaglycerol monocaprate, pentaglycerol monolaurate, hexaglycerol monocaprylate, hexaglycerol monocaprate, hexaglycerol monolaurate, hexaglycerol monomyristate, hexaglycerol monostearate, decaglycerol monocaprylate, decaglycerol monocaprate, decaglycerol monolaurate, decaglycerol monomyristate, decaglycerol monoisostearate, decaglycerol monostearate, decaglycerol monooleate, decaglycerol monohydroxystearate, decaglycerol dicaprylate, decaglycerol dicaprate, decaglycerol dilaurate, decaglycerol dimyristate, decaglycerol diisostearate, decaglycerol distearate, decaglycerol dioleate, decaglycerol dihydroxystearate, decaglycerol tricaprylate, decaglycerol tricaprate, decaglycerol trilaurate, decaglycerol trimyristate, decaglycerol triisostearate, decaglycerol tristearate, decaglycerol trioleate, decaglycerol trihydroxystearate, and mixtures thereof.

The saturated C₁₂ to C₃₀ fatty alcohol emulsifiers are as described in the emollient description set forth above. In one aspect of the invention, the fatty alcohol emulsifier is selected from but not limited to cetyl alcohol, stearyl alcohol, arachidyl alcohol, behenyl alcohol and lanolin alcohol or mixtures of these alcohols, and as are obtainable in the hydrogenation of unsaturated vegetable oil and animal fatty acids.

Emulsifiers based on the esters and partial esters of polyols having 2 to 6 carbon atoms and linear saturated and unsaturated fatty acids having 12 to 30 carbon atoms are, for example, the monoesters and diesters of glycerol or ethylene glycol or the monoesters of propylene glycol with saturated and unsaturated C₁₂ to C₃₀ fatty acids.

The partially esterified polyglycerol emulsifiers include 2 to 10 glycerol units and esterified with 1 to 5 saturated or unsaturated, linear or branched, optionally hydroxylated C₈ to C₃₀ fatty acid residues.

The organosiloxane emulsifiers are polymeric emulsifiers that contain at least one hydrophobic portion and at least one hydrophilic portion. The polymer backbone contains repeating siloxy units that can have cyclic, linear or branched repeating units, e.g. di(C₁ to C₅)alkylsiloxy units, typically dimethylsiloxy units.

The hydrophilic portion of the organosiloxane is generally achieved by substitution onto the polymeric backbone of a residue that confers hydrophilic properties to a portion of the molecule. The hydrophylic residue may be substituted on a terminus of the polymeric organosiloxane, or on any one or more repeating units of the polymer. Generally, the hydrophilic residue is derived from ethylene oxide units that are grafted onto the polymer backbone. In general, the repeating dimethylsiloxy units of modified polydimethylsiloxane emulsifiers are hydrophobic in nature due to the methyl groups, and confer the hydrophobicity properties to the molecule. In addition, longer chain alkyl residues, hydroxy terminated polypropyleneoxy residues, hydroxy terminated polyether residues comprising a combination of ethylene oxide and propylene oxide residues, and/or other types of residues can be substituted onto the siloxy backbone to confer additional emulsification properties to the backbone. Polyether substituted organosiloxane emulsifiers are known as dimethicone copolyols and are widely commercially available. The dimethicone polyols can be random or block copolymers. A generally useful class of dimethicone polyols is block copolymers having blocks of polydimethylsiloxane and blocks of polyalkylene oxide, such as blocks of polyethylene oxide, polypropylene oxide, or both.

### Emollients:

Suitable emollients include but are not limited to an emollient selected from silicone fluids (e.g., volatile silicone oils and non-volatile silicone oils); mineral oils; petrolatums; vegetable oils; fish oils; fatty alcohols; fatty acids; fatty acid and fatty alcohol esters; alkoxylated fatty alcohols; alkoxylated fatty acid esters; benzoate esters; panthenol; Guerbet esters; alkyl ether derivatives of polyethylene glycols, such as, for example methoxypolyethylene glycol (MPEG); and polyalkylene glycols; lanolin and lanolin derivatives; and the like. The emollient can be used alone or in combination with one or more emollients of the present invention.

Volatile silicone oils include cyclic and linear polydimethylsiloxanes, low molecular weight organo-functional silicones, and the like. Cyclic volatile silicones (cyclomethicones) typically contain 3 to 7 silicon atoms, alternating with oxygen atoms, in a cyclic ring structure. Each silicon atom is typically substituted with two alkyl groups, such as, for example, methyl groups. Volatile linear polydimethylsiloxanes (dimethicones) typically contain about 2 to about 9 silicon atoms, alternating with oxygen atoms in a linear arrangement. Each silicon atom is also substituted with two alkyl groups (the terminal silicon atoms are substituted with three alkyl groups), such as, for example, methyl groups. The linear volatile silicones typically have viscosities of less than 5 mPa·s (cP) at 25°C., while the cyclic volatile silicones typically have viscosities of less than 10 mPa·s (cP) at 25°C. "Volatile" means that the silicone has a measurable vapor pressure, or a vapor pressure of at least 267 Pa (2 mm of Hg) at 20°C. Non-volatile silicones have a vapor pressure of less than 267 Pa (2 mm Hg) at 20°C. A description of volatile silicones is found in Todd and Byers, "Volatile Silicone Fluids for Cosmetic Formulations," Cosmetics and Toiletries, Vol. 91, pp. 29 to 32 (January 1976), and in Kasprzak, "Volatile Silicones," Soap/Cosmetics/Chemical Specialties, pp. 40 to 43 (December 1986).

Exemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from G.E. Silicones as SF1173, SF1202, SF1256, and SF1258, Dow Coming Corporation as Dow Corning^{®} 244, 245, 246, 345, and 1401 Fluids. Blends of volatile cyclomethicones and volatile linear dimethicones are also contemplated.

Exemplary volatile linear dimethicones include hexamethyldisiloxane, octamethyltrisiloxane, decamethyltetrasiloxane, dodecamethylpentasiloxane and blends thereof. Volatile linear dimethicones and dimethicone blends are commercially available from Dow Coming Corporation as Dow Corning 200^{®} Fluid (e.g., product designations 0.65 CST, 1°CST, 1.5 CST, and 2 CST) and Dow Corning^{®} 2-1184 Fluid.

Exemplary volatile low molecular weight organo-functional silicones include phenyl trimethicone, caprylyl trimethicone, caprylyl methicone, and hexyl methicene, and blonds thereof. Low molecular weight organe functional silicones are commercially available from Clariant under the trade name Silcare^{®} 41M10, Silcare^{®} 31M60, Silcare^{®} 41M10, and Silcare^{®} 41M15.

The non-volatile silicone oils useful as emollients in the present invention are linear and typically have viscosities of from about 10 mPa·s (cP) to 100,000 mPa·s (cP) at 25°C. They typically contain above 10 dialkyl/diaryl or monoalkyl/monoaryl substituted silicon atoms, alternating with oxygen atoms in a linear arrangement. They include polyalkylsiloxane, polyarylsiloxane, and polyalkylarylsiloxane polymers. Exemplary non-volatile silicone oils include the polydimethylsiloxanes (dimethicones), polydiethylsiloxanes, polymethylphenylsiloxanes, and the like. In one aspect of the invention, the non-volatile silicone oil is selected from a non-volatile polydimethylsiloxane having a viscosity range from 10 mPa·s (cP) to 100,000 mPa·s (cP) at 25°C. Non-volatile dimethicones are commercially available from Dow Corning Corporation as Dow Coming 200^{®} Fluid (product designations 10 CST through 10,000 CST).

Mineral oils and petrolatums include cosmetic, USP and NF grades and are commercially available from Penreco under the Drakeol^{®} and Penreco^{®} trade names. Mineral oil includes hexadecane and paraffin oil.

Exemplary vegetable oils suitable an emollient component in the present invention include but are not limited to peanut oil, sesame oil, avocado oil, coconut oil, cocoa butter, almond oil, safflower oil, com oil, cotton seed oil, sesame seed oil, walnut oil, castor oil, olive oil, jojoba oil, palm oil, palm kernel oil, soybean oil, wheat germ oil, linseed oil, sunflower seed oil; and the mono-, di-, and triglycerides thereof. Exemplary mono-, di- and triglycerides are, for example, caprylic triglyceride, capric triglyceride, caprylic/capric triglyceride, and caprylic/capric/lauric triglyceride, caprylic/capric/stearic triglyceride, and caprylic/capric/linoleic triglyceride.

Ethoxylated mono- and diglycerides are also suitable as an emollient component of the present invention, such as, for example, PEG-8 Caprylic/Capric Glycerides.

Suitable fatty alcohol emollients include but are not limited to fatty alcohols containing 8 to 30 carbon atoms. Exemplary fatty alcohols include capryl alcohol, pelargonic alcohol, capric alcohol, lauryl alcohol, myristyl alcohol, cetyl alcohol, isocetyl alcohol, stearyl alcohol, isostearyl alcohol, cetearyl alcohol, oleyl alcohol, ricinoleyl alcohol, arachidyl alcohol, icocenyl alcohol, behenyl alcohol, and mixtures thereof.

Suitable fatty acid emollients include but are not limited to fatty acids containing 10 to 30 carbon atoms. Exemplary fatty acids are selected from capric acid, lauric acid, myristic acid, palmitic acid, stearic acid, oleic acid, linoleic acid, arachidic acid, behenic acid, and mixtures thereof.

Suitable fatty acid and fatty alcohol ester emollients include but are not limited to hexyl laurate, decyl oleate, isopropyl stearate, isopropyl isostearate, butyl stearate, octyl stearate, cetyl stearate, myristyl myristate, octyldodecyl stearoylstearate, octylhydroxystearate, diisopropyl adipate, isopropyl myristate, isopropyl palmitate, ethyl hexyl palmitate, isodecyl oleate, isodecyl neopentanoate, diisopropyl sebacate, isostearyl lactate, lauryl lactate, diethyl hexyl maleate, PPG-14 butyl ether and PPG-2 myristyl ether propionate, cetearyl octanoate, and mixtures thereof.

Alkoxylated fatty alcohols are ethers formed from the reaction of a fatty alcohol with an alkylene oxide, generally ethylene oxide or propylene oxide. Suitable ethoxylated fatty alcohols are adducts of fatty alcohols and polyethylene oxide. In one aspect of the invention, the ethoxylated fatty alcohols can be represented by the formula R-(OCH₂CH₂)ₙ-OH, wherein R represents the aliphatic residue of the parent fatty alcohol and n represents the number of molecules of ethylene oxide. In another aspect of the invention, R is derived from a fatty alcohol containing 8 to 30 carbon atoms. In one aspect, n is an integer ranging from 2 to 50, 3 to 25 in another aspect, and 3 to 10 in a further aspect. In a still further aspect, R is derived from a fatty alcohol emollient set forth above. Exemplary ethoxylated fatty alcohols are but are not limited to capryl alcohol ethoxylate, lauryl alcohol ethoxylate, myristyl alcohol ethoxylate, cetyl alcohol ethoxylate, stearyl alcohol ethoxylate, cetearyl alcohol ethoxylate oleyl alcohol ethoxylate, and, behenyl alcohol ethoxylate, wherein the number of ethylene oxide units in each of the foregoing ethoxylates can range from 2 and above in one aspect, and from 2 to 150 in another aspect. It is to be recognized that the propoxylated adducts of the foregoing fatty alcohols and mixed ethoxylated/propoxylated adducts of the foregoing fatty alcohols are also contemplated within the scope of the invention. The ethylene oxide and propylene oxide units of the ethoxylated/propoxylated fatty alcohols can be arranged in random or in blocky order.

More specific examples of ethoxylated alcohols are but are not limited to Beheneth 5-30 (the 5-30 meaning the range of repeating ethylene oxide units), Ceteareth 2-100, Ceteth 1-45, Cetoleth 24-25, Choleth 10-24, Coceth 3-10, C9-11 pareth 3-8, C11-15 pareth 5-40, C11-21 Pareth 3-10, C12-13 pareth 3-15, Deceth 4-6, Dodoxynol 5-12, Glycereth 7-26, Isoceteth 10-30, Isodeceth 4-6, Isolaureth 3-6, isosteareth 3-50, Laneth 5-75, Laureth 1-40, Nonoxynol 1-120, Nonylnonoxynol 5-150, Octoxynol 3-70, Oleth 2-50, PEG 4-350, Steareth 2-100, and Trideceth 2-10.

Specific examples of propoxylated alcohols are but are not limited to PPG-10 Cetyl Ether, PPG-20 Cetyl Ether, PPG-28 Cetyl Ether, PPG-30 Cetyl Ether, PPG-50 Cetyl Ether, PPG-2 Lanolin Alcohol Ether, PPG-5 Lanolin Alcohol Ether, PPG-10 Lanolin Alcohol Ether, PPG-20 Lanolin Alcohol Ether, PPG-30 Lanolin Alcohol Ether, PPG-4 Lauryl Ether, PPG-7 Lauryl Ether, PPG-10 Oleyl Ether, PPG-20 Oleyl Ether, PPG-23 Oleyl Ether, PPG-30 Oleyl Ether, PPG-37 Oleyl Ether, PPG-50 Oleyl Ether, PPG-11 Stearyl Ether, PPG-15 Stearyl Ether, PPG-2 Lanolin Ether, PPG-5 Lanolin Ether, PPG-10 Lanolin Ether, PPG-20 Lanolin Ether, PPG-30 Lanolin Ether, and PPG-1 Myristyl Ether.

Specific examples of ethoxylated/propoxylated alcohols are but are not limited to PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-lsodeceth-4, PPG-2-lsodeceth-6, PPG-2-lsodeceth-8, PPG-2-lsodeceth-9, PPG-2-Isodeceth-10, PPG-2-lsodeceth-12, PPG-2-Isodeceth-18, PPG-2-lsodeceth-25, PPG-4-lsodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-Laureth-5, PPG-6-Laureth-3, PPG-25-Laureth-25, PPG-7 Lauryl Ether, PPG-3-Myreth-3, PPG-3-Myreth-11, PPG-20-PEG-20 Hydrogenated Lanolin, PPG-2-PEG-11 Hydrogenated Lauryl Alcohol Ether, PPG-12-PEG-50 Lanolin, PPG-12-PEG-65 Lanolin Oil, PPG-40-PEG-60 Lanolin Oil, PPG-1-PEG-9 Lauryl Glycol Ether, PPG-3-PEG-6 Oleyl Ether, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

Alkoxylated fatty acids are formed when a fatty acid is reacted with an alkylene oxide or with a pre-formed polymeric ether. The resulting product may be a monoester, diester, or mixture thereof. Suitable ethoxylated fatty acid ester emollients suitable for use in the present invention are products of the addition of ethylene oxide to fatty acids. The product is a polyethylene oxide ester of a fatty acid. In one aspect of the invention, the ethoxylated fatty acid esters can be represented by the formula R-C(O)O(CH₂CH₂O)ₙ-H, wherein R represents the aliphatic residue of a fatty acid and n represents the number of molecules of ethylene oxide. In another aspect, n is an integer ranging from 2 to 50, 3 to 25 in another aspect, and 3 to 10 in a further aspect. In still another aspect of the invention, R is derived from a fatty acid containing 8 to 24 carbon atoms. In a still further aspect, R is derived from a fatty acid emollient set forth above. It is to be recognized that propoxylated and ethoxylated/propoxylated products of the foregoing fatty acids are also contemplated within the scope of the invention. Exemplary alkoxylated fatty acid esters include but are not limited to capric acid ethoxylate, lauric acid ethoxylate, myristic acid ethoxylate, stearic acid ethoxylate, oleic acid ethoxylate, coconut fatty acid ethoxylate, and polyethylene glycol 400 propoxylated monolaurate, wherein the number of ethylene oxide units in each of the foregoing ethoxylates can range from 2 and above in one aspect, and from 2 to 50 in another aspect. More specific examples of ethoxylated fatty acids are PEG-8 distearate (the 8 meaning the number of repeating ethylene oxide units), PEG-8 behenate, PEG-8 caprate, PEG-8 caprylate, PEG-8 caprylate/caprate, PEG cocoates (PEG without a number designation meaning that the number of ethylene oxide units ranges from 2 to 50), PEG-15 dicocoate, PEG-2 diisononanoate, PEG-8 diisostearate, PEG-dilaurates, PEG-dioleates PEG-distearates, PEG Ditallates, PEG-isostearates, PEG-jojoba acids, PEG-laurates, PEG-linolenates, PEG-myristates, PEG-oleates, PEG-palmitates, PEG-ricinoleates, PEG-stearates, PEG-tallates, and the like.

Guerbet ester emollients are formed from the esterification reaction of a Guerbet alcohol with a carboxylic acid. Guerbet ester emollients are commercially available from the Noveon Consumer Specialties Division of Lubrizol Advanced Materials, Inc. under product designations G-20, G-36, G-38, and G-66.

Lanolin and lanolin derivatives are selected from lanolin, lanolin wax, lanolin oil, lanolin alcohols, lanolin fatty acids, alkoxylated lanolin, isopropyl lanolate, acetylated lanolin alcohols, and combinations thereof. Lanolin and lanolin derivatives are commercially available from the Noveon Consumer Specialties Division of Lubrizol Advanced Materials, Inc. under the trade names Lanolin LP 108 USP, Lanolin USP AAA, Acetulan™, Ceralan™, Lanocerin™, Lanogel™ (product designations 21 and 41), Lanogene™, Modulan™, Ohlan™, Solulan™ (product designations 16, 75, L-575, 98, and C-24), Vilvanolin™ (product designations C, CAB, L-101, and P).

The emollient(s) can be utilized in an amount ranging from 0.5 weight percent to 30 weight percent based on the total weight of the personal care composition. In another embodiment 0.1 weight percent to 25 weight percent of one or more emollients can be utilized, and in still another embodiment 5 weight percent to 20 weight percent of one or more emollients can be utilized. While emollients are generally employed in personal care compositions, they can be employed in home care, health care, and institutional care compositions in the same weight ratios as set forth for personal care compositions so long as they effect a desired physical attribute (e.g., humectant properties) in such compositions. While overlapping weight ranges for the various components and ingredients that can be contained in the compositions of the invention have been expressed for selected embodiments and aspects of the invention, it should be readily apparent that the specific amount of each component in the disclosed personal care, home care, health care, and institutional care compositions will be selected from its disclosed range such that the amount of each component is adjusted such that the sum of all components in the composition will total 100 weight percent. The amounts employed will vary with the purpose and character of the desired product and can be readily determined by one skilled in the formulation arts and from the literature.

It is also to be recognized that the choice and amount of ingredients in personal care, home care, health care and institutional care compositions that include the surfactant-polymer blend compositions of the invention can vary depending on the intended product and its function, as is well known to those skilled in the formulation arts. An extensive listing of ingredients and their conventional functions and product categories have been disclosed and can be readily ascertained from the literature, some of which can serve more than one function and that one or more of the disclosed ingredients can be combined with the surfactant-polymer compositions to obtain a desired personal care, home care, health care and institutional care composition.

Other oily materials that are useful in combination with the polymers of the present invention include, for example, acetylated lanolin alcohols; lanolin alcohol concentrates; esters of lanolin fatty acids such as the isopropyl esters of lanolin fatty acid; polyol fatty acids; ethoxylated alcohols, such as ethoxylate and castor oils; sterols; sterol esters; sterol ethoxylates; and like materials. Many of such esters and ethoxylates are also useful as non-ionic surfactants.

Numerous ingredients are known in the art as conditioning agents for hair or skin, and humectants, and in addition to those previously discussed, nonlimiting examples include PCA (DL-pyrrolidone carboxylic acid) and its salts, such as lysine PCA, aluminum PCA, copper PCA, chitosan PCA, and the like, allantoin; urea; hyaluronic acid and its salts; ceramides; sorbic acid and its salts; sugars and starches and derivatives thereof; lactamide MEA; and the like.

### Examples

The following examples further illustrate the preparation and use of embodiments. The following examples demonstrate the benefits of using the polymer of this invention as main thickener in a number of surfactant formulations containing a variety of mixtures of anionic to amphoteric surfactants.

### Example 1:

A shampoo formulation is made using sodium laureth sulfate containing 3 moles of ethoxylation (SLES-3EO) and cocamidopropyl betaine. The use of SLES-3EO results in a low eye irritation formulation.

### Example 1 - Formulation

| **Item No.** | **Ingredient Name (Percent Active)** | **Active Weight Percent** | **Weight Percent** |
|---|---|---|---|
| 1a | Sodium laureth sulfate 3EO (30%) | 8 | 26.67 |
| 2a | Cocamidopropyl betaine (35%) | 4 | 11.42 |
| 3a | Acrylates/Beheneth-25 Methacrylate Copolymer* (30%) | 1 | 3.34 |
| 4a | Sodium Chloride | 1 | 1.00 |
| 5a | Sodium Hydroxide (20%) | | Qs to pH 6.5 |
| 6a | Water | | 57.57 |
| | Total | | 100.00 |

| | | | |
|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA). | | | |

The preparation procedure for the above formulation is as follows: (1) at room temperature, in a suitable formulation vessel and under constant mechanical agitation weigh and mix together items 1 a, 2a and 6a until a clear solution is obtained; (2) add Novethix™ L10, item 3a, and mix to obtain a milky liquid; (3) insert a pH meter into the vessel and begin adding item 5a slowly until a max pH of 6.5 is obtained (the solution turns clear and viscous); and (4) add item 4a and mix to homogenize. This results in a clear shampoo formulation with viscosity of 5,150 mPa·s. To this formulation biocide, color, fragrance and other adjuvants can be added as desired. The viscosity of the above formulation is measured with a Brookfield RVT viscometer at 20 rpm, 25°C, Spindle #4-measured after 24 hours.

### Example 2:

A shampoo formulation is made using sodium laureth sulfate containing 2 moles of ethoxylation (SLES-2EO) and cocamidopropyl betaine.

### Example 2 - Formulation

| **Item No.** | **Ingredient Name (Percent Active)** | **Active Weight Percent** | **Weight Percent** |
|---|---|---|---|
| 1b | Sodium laureth sulfate 2EO (26%) | 10 | 38.46 |
| 2b | Cocamidopropyl betaine (35%) | 2 | 14.30 |
| 3b | Acrylates/Beheneth-25 Methacrylate Copolymer* (30%) | 0.5 | 1.67 |
| 4b | Sodium Chloride | 0.5 | 0.5 |
| 5b | Sodium Hydroxide (20%) | | Qs to pH 6.5 |
| 6b | Water | | 46.07 |
| | Total | | 100.00 |

| | | | |
|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) | | | |

The preparation procedure for the above formulation is as follows: (1) at room temperature, in a suitable formulation vessel and under constant mechanical agitation weigh and mix together items 1 b, 2b and 6b until a clear solution is obtained; (2) add Novethix™ L10, item 3b, and mix to obtain a milky liquid; (3) insert a pH meter into the vessel and begin adding item 5b slowly until a max pH of 6.5 is obtained (the solution turns clear and viscous); and (4) add item 4b and mix to homogenize. This results in a clear shampoo formulation with viscosity of 6,900 mPa·s. To this formulation biocide, color, fragrance and other adjuvants can be added as desired. The viscosity of the above formulation is measured with a Brookfield RVT viscometer at 20 rpm, 25°C, Spindle #4-measured after 24 hours.

### Example 3:

A bath gel formulation is made using sodium lauryl sulfate (SLS) and cocobetaine.

### Example 3 - Formulation

| **Item No.** | **Ingredient Name (Percent Active)** | **Active Weight Percent** | **Weight Percent** |
|---|---|---|---|
| 1c | Sodium lauryl sulfate (30%) | 8 | 26.67 |
| 2c | Cocobetaine (43%) | 2 | 4.66 |
| 3c | Acrylates/Beheneth-25 Methacrylate Copolymer* (30%) | 0.5 | 3.34 |
| 4c | Sodium Chloride | 0.5 | 1.0 |
| 5c | Sodium Hydroxide (16%) | | Qs to pH 6.5 |
| 6c | Water | | 64.33 |
| | Total | | 100.00 |

| | | | |
|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) | | | |

The preparation procedure for the above formulation is as follows: (1) at room temperature, in a suitable formulation vessel and under constant mechanical agitation weigh and mix together items 1 c, 2c and 6c until a clear solution is obtained; (2) add Novethix™ L10, item 3c, and mix to obtain a milky liquid; (3) insert a pH meter into the vessel and begin adding item 5c slowly until a max pH of 6.5 is obtained (the solution turns clear and viscous); and (4) add item 4c and mix to homogenize. This results in a clear shampoo formulation with viscosity of 6,900 mPa·s. To this formulation biocide, color, fragrance and other adjuvants can be added as desired. The viscosity of the above formulation is measured with a Brookfield RVT viscometer at 20 rpm, 25°C, Spindle #4-measured after 24 hours.

### Example 4:

This example shows that a blend of the present invention can be used to thicken a number of formulations without the need of salt. A shampoo formulation is made using ammonium laureth sulfate containing 3 moles of ethoxylation (SLES-3EO) and cocamidopropyl betaine.

### Example 4 - Formulation

| **Item No.** | **Ingredient Name (Percent Active)** | **Active Weight Percent** | **Weight Percent** |
|---|---|---|---|
| 1d | Ammonium laurethsulfate 3EO (30%) | 8 | 26.67 |
| 2d | Cocamidopropylbetaine (35%) | 2 | 5.71 |
| 3d | Acrylates/Beheneth-25 Methacrylate Copolymer* (30%) | 1 | 3.34 |
| 4d | 2-Amino-2-methylpropanol | | Qs to pH 6.5 |
| 5d | Water | | 64.28 |
| | Total | | 100.00 |

| | | | |
|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) | | | |

The preparation procedure for the above formulation is as follows: (1) at room temperature, in a suitable formulation vessel and under constant mechanical agitation weigh and mix together items 1d, 2d and 5d until a clear solution is obtained; (2) add Novethix™ L10, item 3d, and mix to obtain a milky liquid; and (3) insert a pH meter into the vessel and begin adding item 4d slowly until a max pH of 6.5 is obtained (the solution turns clear and viscous). This results in a clear shampoo formulation with viscosity of 12,000 mPa·s. To this formulation biocide, color, fragrance and other adjuvants can be added as desired. The viscosity of the above formulation is measured with a Brookfield RVT viscometer at 20 rpm, 25°C, Spindle #4 - measured after 24 hours.

### Example 5:

This example is directed to a two-in-one shampoo and conditioner that utilizes a blend according to an embodiment of the present invention.

### Example 5 - Formulation

| **Part** | | **INCI Name** | **Tradename** | **Weight Percent** | **Supplier** |
|---|---|---|---|---|---|
| A5 | 1 | Deionized Water | | 43.85 | |
| | 2 | Acrylates/Beheneth-25 Methacrylate Copolymer* (30%) | Novethix L-10 | 1.67 | Noveon |
| | 3 | Acrylate Copolymer | Carbopol^{®} Aqua SF-1 Polymer | 2.00 | Noveon |
| | 4 | Sodium Laureth Sulfate (26%) | Texapon HBN | 22.00 | Cognis |
| | 5 | Sodium Hydroxide (18%) | N/A | 0.50 | Synth |
| | 6 | Cocamidopropyl Betaine | Dehyton KB | 2.63 | Cognis |
| B5 | 7 | Cocamide DEA (89%) | Comperlan KD | 2.75 | Cognis |
| | 8 | Glycol Distearate/ Sodium Lauryl Sulfate/Laureth-10/Cocamide MEA | Euperlan PK 810 AM | 3.00 | Cognis |
| C5 | 9 | Deionized Water | | 20.00 | |
| | 10 | Polyquaternium 10 | Celquat SC 240 C | 0.30 | National Starch & Chemical Company |
| D5 | 11 | PEG-7 Glyceryl Cocoate | Cetiol HE | 0.50 | Cognis |
| | 12 | Theobroma Cacau Extract | Coco Extract | 0.20 | Provital S.A |
| | 13 | Fragrance | AVELACHOC O | 0.50 | Firmenich |
| | 14 | Methylchloroisothiazolinon e/ Methylisothiazolinone | Kathon CG | 0.05 | Rohm and Haas Company |
| | 15 | N/A | Sodium Chloride | 0.05 | NORSAL |
| | | Total | | 100.00 | |

| | | | | | |
|---|---|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) | | | | | |

The preparation procedure for the above formulation is as follows: (1) at room temperature, in a suitable formulation vessel and under constant mechanical agitation weigh and mix together items of Part A5 until a clear solution is obtained; (2) in a separate container mix together Part B5 items and heat to 70°C to melt solids, allow to cool to 50°C and add to Part A5; (3) in a separate container, mix items of Part C5 until homogeneous, add Part C5 to the mixture of Parts A5 and B5 above; and (4) in a separate container mix items of Part D5 until homogeneous, add to mixture of Parts A5, B5 and C5.

The product properties of this Example are as follows: appearance-clear liquid; the viscosity is 7,400 mPa·s; pH of 6.48; and the composition is a stable formulation. The viscosity of the above formulation is measured with a Brookfield RVT viscometer at 20 rpm, 25°C, Spindle #4 - measured after 24 hours.

### Example 6:

This example is directed to a two-in-one shampoo and conditioner that utilizes a blend according to an embodiment of the present invention.

### Example 6 - Formulation

| **Part** | | **INCI Name** | **Tradename** | **Weight Percent** | **Supplier** |
|---|---|---|---|---|---|
| A6 | 1 | Deionized Water | | 63.44 | |
| | 2 | Acrylates/Beheneth-25 Methacrylate Copolymer* (30%) | Novethix L-10 | 2.50 | Noveon |
| | 3 | Sodium Laureth Sulfate (26%) | Texapon HBN | 10.00 | Cognis |
| | 4 | Sodium Hydroxide (18%) | N/A | 0.10 | Synth |
| | 5 | Cocamidopropyl Betaine | Dehyton KB | 5.26 | Cognis |
| B6 | 6 | Sodium Laureth Sulfate (26%) | Texapon HBN | 15.00 | Cognis |
| | 7 | Cocamide MIPA | Ninol M-10 | 2.40 | Stepan Company |
| | 8 | PEG-7 Glyceryl Cocoate | Cetiol HE | 0.50 | Cognis |
| | 9 | Deionized Water | | | |
| C6 | 10 | Methol | Menthol | 0.05 | |
| | 11 | Mentha Extract | Extrato de Menta | 0.20 | Provital S.A |
| | 12 | Fragrance | MENTACRE AM | 0.50 | Firmenich |
| D6 | 13 | Methylchloroisothiazolinone/ Methylisothiazolinone | Kathon CG | 0.05 | Rohm and Haas Company |

| | | | | | |
|---|---|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) | | | | | |

The preparation procedure for the above formulation is as follows: (1) at room temperature, in a suitable formulation vessel and under constant mechanical agitation weigh and mix together items of Part A6 until a clear solution is obtained; (2) in a separate container mix together Part B6 items and heat to 70°C to melt solids, allow to cool to 50°C and add to Part A6; (3) in a separate container, mix items of Part C6 until homogeneous, add Part C6 to the mixture of Parts A6 and B6 above; and (4) in a separate container mix items of Part D6 until homogeneous, add to mixture of Parts A6, B6 and C6.

The product properties of this Example are as follows: appearance-clear liquid; the viscosity is 6,300 mPa·s; pH of 6.0; clarity is 9.27 NTU; and the composition is a stable formulation. The viscosity of the above formulation is measured with a Brookfield RVT viscometer at 20 rpm, 25°C, Spindle #4-measured after 24 hours.

### Example 7:

This example is directed to a clear shower gel that is thickened with a blend according to the present invention. This formulation has a low pH and contains no salt. This formulation demonstrates the efficacy of a blend of the present invention to thicken a cleansing formulation containing 6.33 weight percent of total surfactant, using only 1 weight percent of polymer.

### Example 7 - Formulation

| **Item No.** | **Ingredient Name (Percent Active)** | **Weight Percent** |
|---|---|---|
| 1e | Deionized Water | 38.25 |
| 2e | Sodium Laureth Sulfate 2EO (30%) | 16 |
| 3e | Cocamidopropyl betaine (35%) | 3.8 |
| 4e | Cocamide DEA NP | 0.2 |
| 5e | Deionized Water | 32 |
| 6e | Acrylates/Beheneth-25 Methacrylate Copolymer* (30%) | 3.4 |
| 7e | Sodium Chloride (25% in water) | 4 |
| 8e | DMDM Hydantoin | 0.1 |
| 9e | Strawberry Fragrance | 0.5 |
| 10e | FD&C Red 40 | 0.8 |
| 11e | Sodium hydroxide (18%) | 0.95 |
| | Total | 100.00 |

| | | |
|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) | | |

The preparation procedure for the above formulation is as follows: (1) at room temperature in a suitable formulation vessel, under mechanical agitation, mix items 1e, 2e, 3e and 4e together; (2) add item 6e under constant agitation; (3) add items 7e, 8e, 9e, 10e and mix to homogenize; and (4) add item 11 e to adjust the formulation's pH to 6.6.

The product properties of this Example are as follows: appearance-clear elegant red fluid; the viscosity is 6,400 mPa·s; and a pH of 6.6. The viscosity of the above formulation is measured with a Brookfield DV-II+ Pro viscometer at 20 rpm, 25°C, Spindle #6.

### Hair Styling Gel Examples 8 Through 10:

The following examples demonstrate the benefits of using a blend of the present invention as a main thickener in a hair styling gel composition.

### Examples 8 Through 10 - Formulations

| **Item No.** | **Ingredient Name (Percent Active)** | **Example 8 Weight Percent** | **Example 9 Weight Percent** | **Example 10 Weight Percent** |
|---|---|---|---|---|
| 1f | Water | 94.50 | 98.00 | 95.25 |
| 2f | Acrylates/Beheneth-25 Methacrylate Copolymer* | 0.50 | 0.50 | 0.50 |
| 3f | Polyacrylate-2 Crosspolymer** | 4.00 | | |
| 4f | Polyacrylate-14*** | | 0.5 | |
| | PVP K90 | | | 3.00 |
| | Laureth-30 | | | 0.25 |
| 5f | Glydant Plus | 1.00 | 1.00 | 1.00 |
| 6f | AMP95 | Qs to pH 6.8 | Qs to pH 6.8 | qs |
| | | | | |
| | pH at 25°C | 6.8 - 7.0 | 6.8 - 7.0 | 6.8 - 7.0 |
| | Viscosity at 25°C | 38,000 | 23,000 | 20,000 |
| | Clarity | 84% | 83% | 89% |

| | | | | |
|---|---|---|---|---|
| * INCI name for polymer component (c) of the present invention. The trade name is Novethix™ L10 Polymer (Noveon Consumer Specialties segment of Lubrizol Advanced Materials, Inc., Cleveland OH, USA) ** This is a hair fixative polymer sold under the trade name Fixate™ Superhold Polymer (Noveon Consumer Specialties segment of Lubrizol Advanced Materials, Inc., Cleveland OH, USA). *** This is a hair fixative polymer sold under the trade name Fixate™ Plus Polymer (Noveon Consumer Specialties segment of Lubrizol Advanced Materials, Inc., Cleveland OH, USA). | | | | |

The viscosity is measured with a Brookfield RVT viscometer at 20 rpm, 25°C, Spindle #6, measured after 24 hours. The clarity is measured with an HF Scientific, Inc., Micro 100 Turbidimeter. Glydant Plus is a biocide sold by Lonza Inc, NJ 07401, USA. AMP95 is aminomethyl propanol sold by the Angus Co, USA.

### Hair Gel Preparation Procedure:

The preparation procedure for each of Examples 8 through 10 is as follows. In a formulation vessel, at room temperature, weigh items 1f, 2f and 6f and mix thoroughly to obtain a clear viscous gel; add items 4f or 5f while mixing; and then add 6f. Fragrance and color can optionally be added.

### Sulfate-Free Examples 11 Through 13:

The following Examples 11 through 13 demonstrate the use of a blend of the present invention as an effective thickener in sulfate-free cleansing compositions.

### Example 11 - Sulfate-Free Bath Gel Cleanser:

This formulation is cold processable, mild and gentle sulfate-free bath gel cleanser effectively thickened with 1.0 weight percent of a blend according to the present invention.

### Example 11 - Formulation

| **Item No.** | **Tradename - Supplier** | **INCI Name (Percent Active)** | **Weight Percent** | **Function** |
|---|---|---|---|---|
| 1g | Deionized Water | Deionized Water | 50.000% | Diluent |
| 2g | Jordapon ACI-30-G - BASF | Ammonium Cocoyl Isethionate (30%) | 6.667% | Surfactant |
| 3g | Chemoryl SFB-10 SK-Chemron | Disodium Laureth Sulfosuccinate & Sodium Cocyl Isethionate & Cocamidopropyl Betaine (32%) | 17.969% | Surfactant |
| 4g | Deionized Water | Deionized Water | 8.178% | Diluent |
| 5g | Novethix L-10-Lubrizol | Acrylates/Beheneth-25 Methacrylate Copolymer (30%) | 3.334% | Rheology Modifier |
| 6g | Phenonip ME-Clariant | Phenoxyethanol (and) Methylparaben (and) Ethylparaben | 0.500% | Preservative |
| 7g | Chemccinate LHS - Chemron | Lauryl Hydroxysultaine (49.9%) | 4.010% | Surfactant |
| 8g | Chemoryl LB-30 - Chemron | Oleyl Betaine & Sodium Lauroyl Lactylate (18%) | 5.560% | Surfactant |
| 9g | Schercoteric MS-2-50 - Chemron | Disodium Cocoamphodiacetate (45%) | 2.779% | Surfactant |
| 10g | NaOH | Sodium Hydroxide (18%) | 0.900% | Neutralizer |
| 11g | Fragrance | Fragrance | 0.100% | Perfuming Agent |
| | Total | | 100.0% | |

The product properties of this Example are as follows: appearance-clear; the viscosity is 15,000 ± 2,000 mPa·s; a pH of 6.4 to 6.6; turbidity 18.1 NTU; and the formulations passes 10 weeks at 45°C and 5 freeze/thaw cycles. The viscosity is measured with a Brookfield RVT viscometer at 20 rpm, 25°C, Spindle #6, measured after 24 hours. The clarity is measure with an HF Scientific, Inc., Micro 100 Turbidimeter.

The preparation procedure for the above formulation is as follows: (1) at room temperature, in a suitable formulation vessel and under constant mechanical agitation, weigh and mix items 1g, 2g and 3g until a clear solution is obtained; (2) add items 4g and 5g, and mix to obtain a milky liquid; (3) insert a pH meter into the vessel and begin adding item 10g slowly until a max pH of 6.7 is obtained (the solution turns clear and viscous); (4) add items 6g, 7g, 8g and 9g in sequential numerical order; and (5) add item 11 g and mix gently to homogenize.

### Example 12 - Sulfate-Free Body Wash:

This formulation is cold processable, mild and gentle sulfate-free body wash cleanser effectively thickened with 1.0 weight percent of a blend according to the present invention.

### Example 12 - Formulation

| **Item No.** | **Tradename - Supplier** | **INCI Name (Percent Active)** | **Weight Percent** | **Function** |
|---|---|---|---|---|
| 1h | Deionized Water | Deionized Water | 50.000% | Diluent |
| 2h | Jordapon ACI-30-G - BASF | Ammonium Cocoyl Isethionate (30%) | 5.000% | Surfactant |
| 3h | Chemcinnate DSLS - Chemron | Disodium Laureth Sulfosuccinate (40%) | 5.000% | Surfactant |
| 4h | Deionized Water | Deionized Water | 10.962% | Diluent |
| 5h | Novethix™ L10-Lubrizol Advanced Materials Inc. | Acrylates/Beheneth-25 Methacrylate Copolymer (30%) | 3.334% | Rheology Modifier |
| 6h | Chemccinate LHS - Chemron | Lauryl Hydroxysultaine (49.9%) | 7.016% | Surfactant |
| 7h | Kathon CG - Rohm & Haas | Methylchloroisothiazolinone and Methylisothiazolinone | 0.050% | Preservative |
| 8h | Chemoryl LB-30-Chemron | Oleyl Betaine & Sodium Lauroyl Lactylate (18%) | 8.340% | Surfactant |
| 9h | Chembetaine CAD - Chemron | Cocomidopropyl Betaine (35%) | 6.429% | Amphoteric Surfactant |
| 10h | Schercoteric MS-2-50 - Chemron | Disodium Cocoamphodiacetate (45%) | 2.779% | Surfactant |
| 11h | NaOH | Sodium Hydroxide (18%) | 0.990% | Neutralizer |
| 12h | Fragrance | | 0.100% | Perfuming Ingredient |
| | Total | | 100.0% | |

The product properties of this Example are as follows: appearance-clear to slightly hazy; the viscosity is 8,160 ± 1,000 mPa·s; a pH of 6.4 to 6.6; turbidity 31.9 NTU; and the formulations passes 10 weeks at 45°C and 5 freeze/thaw cycles. The viscosity is measured with a Brookfield RVT viscometer at 20 rpm, 25°C, Spindle #5, measured after 24 hours. The clarity is measure with an HF Scientific, Inc., Micro 100 Turbidimeter.

The preparation procedure for the above formulation is as follows: (1) at room temperature, in a suitable formulation vessel and under constant mechanical agitation, weight and mix items 1 h, 2h and 3h until a clear solution is obtained; (2) add items 4h and 5h, and mix to obtain a milky liquid; (3) insert a pH meter into the vessel and begin adding item 11 h slowly until a max pH of 6.7 is obtained (the solution turns clear and viscous); (4) add items 6h, 7h, 8h, 9h and 10h in sequential numerical order; and (5) add item 12h and mix gently to homogenize.

### Example 13 - Sulfate-Free Shampoo Formulation:

This formulation is sulfate-free shampoo formulation effectively thickened with 0.75 weight percent of a blend according to the present invention.

### Example 13 - Formulation

| **Item No.** | **Tradename - Supplier** | **INCI Name (Percent Active)** | **Weight Percent** | **Function** |
|---|---|---|---|---|
| 1j | Deionized Water | Deionized Water | 50.000% | Diluent |
| 2j | Jordapon ACI-30-G - BASF | Ammonium Cocoyl Isethionate (30%) | 6.667% | Surfactant |
| 3j | Chemoryl SFB-10 SK-Chemron | Disodium Laureth Sulfosuccinate & Sodium Cocyl Isethionate & Cocomidopropyl Betaine (32%) | 17.969% | Surfactant |
| 4j | Deionized Water | Deionized Water | 9.135% | Diluent |
| 5j | Novethix™ L10 - Lubrizol Advanced Materials Inc. | Acrylates/Beheneth-25 Methacrylate Copolymer (30%) | 2.500% | Rheology Modifier |
| 6j | Phenonip ME - Clariant | Phenoxyethanol (and) Methylparaben (and) Ethylparaben | 0.500% | Preservative |
| 7j | Chemccinate LHS - Chemron | Lauryl Hydroxysultaine (49.9%) | 4.010% | Surfactant |
| 8j | Chemoryl LB-30 - Chemron | Oleyl Betaine & Sodium Lauroyl Lactylate (18%) | 5.560% | Surfactant |
| 9j | Schercoteric MS-2-50-Chemron | Disodium Cocoamphodiacetate (45%) | 2.779% | Surfactant |
| 10j | Fragrance | Fragrance | 0.1% | Perfuming agent |
| 11j | NaOH | Sodium Hydroxide (18%) | 0.780% | Neutralizer |
| | Total | | 100% | |

The product properties of this Example are as follows: appearance-clear to slightly hazy; the viscosity is 6,280 mPa·s; a pH of 6.4 to 6.6; turbidity 32.5 NTU; and the formulations passes 10 weeks at 45°C and 5 freeze/thaw cycles. The viscosity is measured with a Brookfield RVT viscometer at 20 rpm, 25°C, Spindle #5, measured after 24 hours. The clarity is measure with an HF Scientific, Inc., Micro 100 Turbidimeter.

The preparation procedure for the above formulation is as follows: (1) at room temperature, in a suitable formulation vessel and under constant mechanical agitation, weight and mix items 1 j, 2j, 3j and 34 until a clear solution is obtained; (2) add item 5j, and mix to obtain a milky liquid; (3) insert a pH meter into the vessel and begin adding item 11j slowly until a pH of 6.5 to 6.7 is obtained (the solution turns clear and viscous); and (4) add items 6j and 10j.

## Claims

1. A surfactant-polymer blend comprising:
(a) at least one anionic surfactant;
(b) at least one amphoteric or zwitterionic surfactant;
(c) at least one hydrophobically-modified polymer formed from a mixture comprising:
(i) at least one α,β-ethylenically unsaturated carboxylic acid monomer selected from acrylic acids, methacrylic acids, crotonic acids, acyloxypropionic acids, maleic acids, fumaric acids, itaconic acids, aconitic acids, half- or mono-esters of dibasic or tribasic acids;
(ii) at least one nonionic, co-polymerizable α,β-ethylenically unsaturated monomer selected from a C₁ to C₄ alkyl acrylate, C₁ to C₄ alkyl methacrylate; and
(iii) at least one ethylenically unsaturated hydrophobically-modified associative monomer selected from cetyl polyethoxylated (meth)acrylate (CEM), cetearyl polyethoxylated (meth)acrylate (CSEM), stearyl polyethoxylated (meth)acrylate, arachidyl polyethoxylated (meth)acrylate, behenyl polyethoxylated methacrylate (BEM), cerotyl polyethoxylated (meth)acrylate, montanyl polyethoxylated (meth)acrylate, melissyl polyethoxylated (meth)acrylate, lacceryl polyethoxylated (meth)acrylate, nonylphenol polyethoxylated (meth)acrylate (NEM), tristyrylphenol polyethoxylated (meth)acrylate (TEM), palmyl polyethoxylated itaconate (PEI), stearyl polyethoxylated itaconate (SEI), behenyl polyethoxylated itaconate (BEI), palmyl polyethoxylated maleate (PEML), stearyl polyethoxylated maleate (SEM), behenyl polyethoxylated maleate (BEML), palmyl polyethoxylated allyl ether (PEAE), stearyl polyethoxylated allyl ether (SEAE), behenyl polyethoxylated allyl ether (BEAE), wherein the polyethoxylated portion of the foregoing monomers contain from 3 to 200 ethylene oxide repeating unit; and
(d) at least one monovalent salt selected from lithium chloride, sodium chloride, ammonium chloride, potassium chloride, sodium benzoate, sodium salicylate or mixtures of any two or more thereof.

2. The surfactant-polymer blend of claim 1, wherein the weight ratio of the combination of components (a) and (b) to component (c) is in the range of from 2500:1 to 5:1.

3. The surfactant-polymer blend of claim 1, wherein the amounts of components (i), (ii) and (iii) that are utilized in a suitable polymerization reaction to produce the at least one hydrophobically-modified polymer of component (c) are as follows:
(i) from 2.5 weight percent to 75 weight percent of at least one α,β-ethylenically unsaturated carboxylic acid monomer;
(ii) from 0 weight percent to 90 weight percent of at least one nonionic, co-polymerizable α,β-ethylenically unsaturated monomer; and
(iii) from 0.5 weight percent to 40 weight percent of at least one ethylenically unsaturated hydrophobically-modified associative monomer.

4. The surfactant-polymer blend of claim 1, wherein the blend further comprises one or more non-ionic surfactants, one or more cationic surfactants, one or more amide surfactants, or a mixture of two or more thereof.

5. The surfactant-polymer blend of claim 1, wherein the blend further comprises one or more cationic polymers, one or more multi-purpose polymers, one or more compounds that enhancing performance or consumer acceptability, or mixtures of two or more thereof.

6. The surfactant-polymer blend of claim 1, wherein the weight ratio of the combination of components (a) and (b) to component (c) to component (d) is in the range of 2500:1:1 to 5:1:1.

7. The surfactant polymer blend of claim 1, wherein component (a) is selected from alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, alkyl ether carboxylic acids, and mixtures thereof.

8. The surfactant polymer blend of claim 7, wherein component (a) is selected from sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

9. The surfactant polymer blend of claim 1, wherein component (b) is selected from alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates, acyl glutamates, wherein the alkyl and acyl groups have from 8 to 19 carbon atoms.

10. The surfactant polymer blend of claim 1, wherein component (b) is selected from lauryl amine oxide, cocodimethyl sulphopropyl betaine, lauryl betaine, cocamidopropyl betaine, and sodium cocoamphoacetate, cocoamidopropyl betaine (CAPB), cocobetaine,
and mixtures thereof.

11. A personal care product, a health care product, a household care product, an institutional or an industrial care product comprising the surfactant-polymer blend of any one of claims 1 to 10.

## Patentansprüche

1. Tensid-Polymer-Gemisch, umfassend:
(a) wenigstens ein anionisches Tensid;
(b) wenigstens ein amphoteres oder zwitterionisches Tensid;
(c) wenigstens ein hydrophob modifiziertes Polymer, das aus einem Gemisch gebildet ist, welches Folgendes umfasst:
(i) wenigstens ein α,β-ethylenisch ungesättigtes Carbonsäuremonomer, das aus Acrylsäuren, Methacrylsäuren, Krotonsäuren, Acyloxypropionsäuren, Maleinsäuren, Fumarsäuren, Itaconsäuren, Aconitsäuren, Halb- oder Monoestern von zweibasigen oder dreibasigen Säuren ausgewählt ist;
(ii) wenigstens ein nichtionisches copolymerisierbares, α,β-ethylenisch ungesättigtes Monomer, das aus einem C₁- bis C₄-Alkylacrylat, C₁- bis C₄-Alkylmethacrylat ausgewählt ist; und
(iii) wenigstens ein ethylenisch ungesättigtes, hydrophob modifiziertes assoziatives Monomer, das aus Cetyl-polyethoxyliertem-(meth)acrylat (CEM), Cetearyl-polyethoxyliertem-(meth)acrylat (CSEM), Stearyl-polyethoxyliertem-(meth)-acrylat, Arachidyl-polyethoxyliertem-(meth)acrylat, Behenyl-polyethoxyliertem-methacrylat (BEM), Cerotyl-polyethoxyliertem-(meth)acrylat, Montanyl-polyethoxyliertem-(meth)-acrylat, Melissyl-polyethoxyliertem-(meth)acrylat, Lacceryl-polyethoxyliertem-(meth)acrylat, Nonylphenol-polyethoxy-liertem-(meth)acrylat (NEM), Tristyrylphenol-polyethoxylier-tem-(meth)acrylat (TEM), Palmyl-polyethoxyliertem-itaconat (PEI), Stearyl-polyethoxyliertem-itaconat (SEI), Behenyl-polyethoxyliertem-itaconat (BEI), Palmyl-polyethoxyliertemmaleat (PEML), Stearyl-polyethoxyliertem-maleat (SEM), Behenyl-polyethoxyliertem-maleat (BEML), Palmyl-polyethoxyliertem-allylether (PEAE), Stearyl-polyethoxyliertem-allylether (SEAE), Behenyl-polyethoxyliertem-allylether (BEAE) ausgewählt ist, wobei der polyethoxylierte Teil der obigen Monomere 3 bis 200 Ethylenoxid-Repetiereinheiten enthält; und
(d) wenigstens ein einwertiges Salz, das aus Lithiumchlorid, Natriumchlorid, Ammoniumchlorid, Kaliumchlorid, Natriumbenzoat, Natriumsalicylat oder Gemischen von zweien oder mehreren davon ausgewählt ist.

2. Tensid-Polymer-Gemisch gemäß Anspruch 1, wobei das Gewichtsverhältnis der Kombination der Komponenten (a) und (b) zu Komponente (c) im Bereich von 2500:1 bis 5:1 liegt.

3. Tensid-Polymer-Gemisch gemäß Anspruch 1, wobei die Mengen der Komponenten (i), (ii) und (iii), die in einer geeigneten Polymerisationsreaktion zur Herstellung des wenigstens einen hydrophob modifizierten Polymers der Komponente (c) verwendet werden, wie folgt sind:
(i) 2,5 Gew.-% bis 75 Gew.-% wenigstens eines α,β-ethylenisch ungesättigten Carbonsäuremonomers;
(ii) 0 Gew.-% bis 90 Gew.-% wenigstens eines nichtionischen copolymerisierbaren, α,β-ethylenisch ungesättigten Monomers; und
(iii) 0,5 Gew.-% bis 40 Gew.-% wenigstens eines ethylenisch ungesättigten, hydrophob modifizierten assoziativen Monomers.

4. Tensid-Polymer-Gemisch gemäß Anspruch 1, wobei das Gemisch weiterhin ein oder mehrere nichtionische Tenside, ein oder mehrere kationische Tenside, ein oder mehrere Amid-Tenside oder ein Gemisch von zweien oder mehreren davon umfasst.

5. Tensid-Polymer-Gemisch gemäß Anspruch 1, wobei das Gemisch weiterhin ein oder mehrere kationische Polymere, ein oder mehrere Mehrzweckpolymere, eine oder mehrere Verbindungen, die die Leistungsfähigkeit oder Verbraucherakzeptanz erhöhen, oder Gemische von zweien oder mehreren davon umfasst.

6. Tensid-Polymer-Gemisch gemäß Anspruch 1, wobei das Gewichtsverhältnis der Kombination der Komponenten (a) und (b) zu Komponente (c) zu Komponente (d) im Bereich von 2500:1:1 bis 5:1:1 liegt.

7. Tensid-Polymer-Gemisch gemäß Anspruch 1, wobei Komponente (a) aus Alkylsulfaten, Alkylethersulfaten, Alkarylsulfonaten, Alkanoylisethionaten, Alkylsuccinaten, Alkylsulfosuccinaten, Alkylethersulfosuccinaten, N-Alkylsarcosinaten, Alkylphosphaten, Alkyletherphosphaten und Alkylethercarbonsäuren ausgewählt ist.

8. Tensid-Polymer-Gemisch gemäß Anspruch 7, wobei Komponente (a) aus Natriumoleylsuccinat, Ammoniumlaurylsulfosuccinat, Natriumlaurylsulfat, Natriumlaurylethersulfat, Natriumlaurylethersulfosuccinat, Ammoniumlaurylsulfat, Ammoniumlaurylethersulfat, Natriumdodecylbenzolsulfonat, Triethanolamindodecylbenzolsulfonat, Natriumcocoylisethionat, Natriumlaurylisethionat, Laurylethercarbonsäure und Natrium-N-laurylsarcosinat ausgewählt ist.

9. Tensid-Polymer-Gemisch gemäß Anspruch 1, wobei Komponente (b) aus Alkylaminoxiden, Alkylbetainen, Alkylamidopropylbetainen, Alkylsulfobetainen (Sultainen), Alkylglycinaten, Alkylcarboxyglycinaten, Alkylamphoacetaten, Alkylamphopropionaten, Alkylamphoglycinaten, Alkylamidopropylhydroxysultainen, Acyltauraten und Acylglutamaten ausgewählt ist, wobei die Alkyl- und Acylgruppen 8 bis 19 Kohlenstoffatome aufweisen.

10. Tensid-Polymer-Gemisch gemäß Anspruch 1, wobei Komponente (b) aus Laurylaminoxid, Cocodimethylsulfopropylbetain, Laurylbetain, Cocamidopropylbetain und Natriumcocoamphoacetat, Cocoamidopropylbetain (CAPB), Cocobetain und Gemischen davon ausgewählt ist.

11. Körperpflegeprodukt, Hygieneprodukt, Haushaltspflegeprodukt, institutionelles oder industrielles Pflegeprodukt, das das Tensid-Polymer-Gemisch gemäß einem der Ansprüche 1 bis 10 umfasst.

## Revendications

1. Mélange de polymère-tensioactif comprenant :
(a) au moins un tensioactif anionique ;
(b) au moins un tensioactif amphotère ou zwittérionique ;
(c) au moins un polymère modifié sur le plan hydrophobe, formé d'un mélange comprenant :
(i) au moins un monomère d'acide carboxylique α,β-éthyléniquement insaturé choisi parmi les acides acryliques, acides méthacryliques, acides crotoniques, acides acyloxypropioniques, acides maléiques, acides fumariques, acides itaconiques, acides aconitiques, semi- ou mono-esters d'acides dibasiques ou tribasiques ;
(ii) au moins un monomère non ionique, copolymérisable, α,β-éthyléniquement insaturé choisi parmi un acrylate d'alkyle en C₁ à C₄, un méthacrylate d'alkyle en C₁ à C₄ ; et
(iii) au moins un monomère associatif éthyléniquement insaturé, modifié sur le plan hydrophobe choisi parmi le (méth)acrylate de cétyle polyéthoxylé (CEM), le (méth)acrylate de cétéaryle polyéthoxylé (CSEM), le (méth)acrylate de stéaryle polyéthoxylé, le (méth)acrylate d'arachidyle polyéthoxylé, le méthacrylate de béhényle polyéthoxylé (BEM), le (méth)acrylate de cérotyle polyéthoxylé, le (méth)acrylate de montanyle polyéthoxylé, le (méth)acrylate de mélissyle polyéthoxylé, le (méth)acrylate de laccéryle polyéthoxylé, le (méth)acrylate de nonylphénol polyéthoxylé (NEM), le (méth)acrylate de tristyrylphénol polyéthoxylé (TEM), l'itaconate de palmyle polyéthoxylé (PEI), l'itaconate de stéaryle polyéthoxylé (SEI), l'itaconate de béhényle polyéthoxylé (BEI), le maléate de palmyle polyéthoxylé (PEML), le maléate de stéaryle polyéthoxylé (SEM), le maléate de béhényle polyéthoxylé (BEML), l'allyléther de palmyle polyéthoxylé (PEAE), l'allyléther de stéaryle polyéthoxylé (SEAE), l'allyléther de béhényle polyéthoxylé (BEAE), dans lequel la portion polyéthoxylée des monomères précédents contient de 3 à 200 motifs répétitifs d'oxyde d'éthylène ; et
(d) au moins un sel monovalent choisi parmi le chlorure de lithium, le chlorure de sodium, le chlorure d'ammonium, le chlorure de potassium, le benzoate de sodium, le salicylate de sodium ou des mélanges de deux quelconques ou plus de ceux-ci.

2. Mélange de polymère-tensioactif selon la revendication 1, dans lequel le rapport en poids de la combinaison de composants (a) et (b) au composant (c) est dans la plage de 2500 : 1 à 5 : 1.

3. Mélange de polymère-tensioactif selon la revendication 1, dans lequel les quantités de composants (i), (ii) et (iii) qui sont utilisées dans une réaction de polymérisation appropriée pour produire l'au moins un polymère modifié sur le plan hydrophobe du composant (c) sont les suivantes :
(i) de 2,5 % en poids à 75 % en poids d'au moins un monomère d'acide carboxylique α,β-éthyléniquement insaturé ;
(ii) de 0 % en poids à 90 % en poids d'au moins un monomère non ionique, copolymérisable, α,β-éthyléniquement insaturé ; et
(iii) de 0,5 % en poids à 40 % en poids d'au moins un monomère associatif éthyléniquement insaturé, modifié sur le plan hydrophobe.

4. Mélange de polymère-tensioactif selon la revendication 1, dans lequel le mélange comprend en outre un ou plusieurs tensioactifs non ioniques, un ou plusieurs tensioactifs cationiques, un ou plusieurs tensioactifs amides, ou un mélange de deux ou plus de ceux-ci.

5. Mélange de polymère-tensioactif selon la revendication 1, dans lequel le mélange comprend en outre un ou plusieurs polymères cationiques, un ou plusieurs polymères polyvalents, un ou plusieurs composés qui améliorent la performance ou l'acceptabilité par le consommateur, ou des mélanges de deux ou plus de ceux-ci.

6. Mélange de polymère-tensioactif selon la revendication 1, dans lequel le rapport en poids de la combinaison des composants (a) et (b) au composant (c) au composant (d) est dans la plage de 2500 : 1 : 1 à 5 : 1 : 1.

7. Mélange de polymère-tensioactif selon la revendication 1, dans lequel le composant (a) est choisi parmi les sulfates d'alkyle, les sulfates d'alkyléther, les sulfonates d'alcaryle, les iséthionates d'alcanoyle, les succinates d'alkyle, les sulfosuccinates d'alkyle, les sulfosuccinates d'alkyléther, les sarcosinates de N-alkyle, les phosphates d'alkyle, les phosphates d'lkyléther, les acides carboxyliques d'alkyléther et leurs mélanges.

8. Mélange de polymère-tensioactif selon la revendication 7, dans lequel le composant (a) est choisi parmi l'oléylsuccinate de sodium, le laurylsulfosuccinate d'ammonium, le laurylsulfate de sodium, le lauryléthersulfate de sodium, le lauryléthersulfosuccinate de sodium, le laurylsulfate d'ammonium, le lauryléthersulfate d'ammonium, le dodécylbenzènesulfonate de sodium, le dodécylbenzènesulfonate de triéthanolamine, le cocoyliséthionate de sodium, le lauryliséthionate de sodium, l'acide de lauryléther carboxylique et le N-lauryl-sarcosinate de sodium.

9. Mélange de polymère-tensioactif selon la revendication 1, dans lequel le composant (b) est choisi parmi les oxydes d'alkylamine, les alkylbétaïnes, les alkylaminopropylbétaïnes, les alkylsulfobétaïnes (sultaïnes), les glycinates d'alkyle, les carboxyglycinates d'alkyle, les ampho-acétates d'alkyle, les amphopropionates d'alkyle, les amphoglycinates d'alkyle, les alkylaminopropyl-hydroxysultaïnes, les taurates d'acyle, les glutamates d'acyle, dans lequel les groupes alkyle et acyle ont de 8 à 19 atomes de carbone.

10. Mélange de polymère-tensioactif selon la revendication 1, dans lequel le composant (b) est choisi parmi l'oxyde de laurylamine, la cocodiméthylsulfopropylbétaïne, la laurylbétaïne, la cocamidopropylbétaïne et le coco-ampho-acétate de sodium ; la coco-amidopropyl-bétaïne (CAPB), la cocobétaïne et leurs mélanges.

11. Produit de toilette personnel, produit de soin de santé, produit d'entretien domestique, produit d'entretien dans le cadre institutionnel ou industriel, comprenant le mélange de polymère-tensioactif selon l'une quelconque des revendications 1 à 10.
